Europäisches Patentamt

European Patent Office

Office européen des brevets

(19)

(11) Publication number: **0 270 077**

**A2**

(12)

# EUROPEAN PATENT APPLICATION

(21) Application number: 87117760.6

(22) Date of filing: 01.12.87

(51) Int. Cl.⁴: **C12N 15/00** , C12N 1/20 , C12P 21/02 , C07K 15/06 , C12P 21/00 , C12N 5/00

The applicant has filed a statement in accordance with Rule 28 (4) EPC (issue of a sample only to an expert). Accession number(s) of the deposit(s): Ferm BP-1548, Ferm BP-1550, Ferm BP-1549.

(30) Priority: 03.12.86 JP 288340/86
26.11.87 JP 298513/87

(43) Date of publication of application:
08.06.88 Bulletin 88/23

(84) Designated Contracting States:
AT BE CH DE FR GB IT LI NL SE

(71) Applicant: SUMITOMO CHEMICAL COMPANY, LIMITED
15 Kitahama 5-chome Higashi-ku
Osaka-shi Osaka 541(JP)

Applicant: SUMITOMO PHARMACEUTICALS COMPANY, LIMITED
40, Dosho-machi 2-chome
Higashi-ku Osaka-shi Osaka-fu(JP)

(72) Inventor: Nakatani, Tomoyuki
1-23-6, Yuyamadai
Kawanishi-shi Hyogo-ken(JP)
Inventor: Nomura, Noriko
7-1-21, Midoridai
Kawanishi-shi Hyogo-ken(JP)
Inventor: Horigome, Kazuhiko
2-14-7, Mefu
Takarazuka-shi Hyogo-ken(JP)
Inventor: Noguchi, Hiroshi
4-4-153, Seiwadai-nishi
Kawanishi-shi Hyogo-ken(JP)

(74) Representative: Vossius & Partner
Siebertstrasse 4 P.O. Box 86 07 67
D-8000 München 86(DE)

(54) Antibody to exotoxin of pseudomonas aeruginosa and gene therefor.

(57) A gene encoding an antibody to an exotoxin of Pseudomonas aeruginosa, a recombinant plasmid or virus containing said gene, and a transformed host cell harboring said recombinant plasmid or virus are provided. A process for preparing human monoclonal antibody to the exotoxin of Pseudomonas aeruginosa and the prepared monoclonal antibody by said process are also provided.

EP 0 270 077 A2

## Antibody to Exotoxin of Pseudomonas aeruginosa and Gene therefor

The present invention relates to a gene encoding an antibody to an exotoxin of Pseudomonas aeruginosa, a recombinant plasmid harboring the gene, and a transformant containing the recombinant · plasmid. More particularly, the invention relates to a genomic DNA encoding the antibody, which has been obtained from a cell producing monoclonal antibody to the exotoxin. a corresponding cDNA, a recombinant plasmid harboring said genomic DNA or cDNA, a transformant containing said recombinant plasmid, a method for the production of the antibody, which process comprises culturing said transformant under appropriate conditions, and the antibody produced by said method.

P. aeruginosa infection is one of major medical problems because it often causes serious conditions. particularly in patients suffering from immuno deficiency or immune depression. One of the major pathogenic substances responsible for the serious symptom of the disease is a variety of virulent factors produced and secreted by P . aeruginosa. The exotoxin inhibits biosynthesis of proteins through ADP-ribosylation of a polypeptide chain elongation factor, which results in cytotoxicity, necrosis of tissues, and eventual death of the patients. Antibiotics often used for the treatment of various bacterial infections are not valid to the exotoxin. However, antibody therapy is expected to be useful for combating the exotoxin.

Extended studies have been done on structure and function of a wide range of antibodies, i.e., immunoglobulins (See, for instance, Kabat, E.A., Structural Concepts in Immunology and Immunochemistry. 2nd ed., Holt, Rinehart and Winston, New York, London, 1976).

Antibody is an antigen-specific glycoprotein which is produced by B lymphocyte through sensitization with the antigen, which is a foreign substance invaded into a living body. Human immunoglobulins are divided into five classes, i.e., IgG, IgM, IgA, IgD; and IgE. IgG consists of a pair of light polypeptide chains (L chain) having a molecular weight of 25,000 and a pair of heavy polypeptide chains (H chain) having a molecular weight of 51,000. These chains are usually linked by disulfide bonds between H and L chains and between two H chains. A sequence comprising about 100 amino acid residues at N-terminal of each of H and L chains is specific and unique to a given antigen and constitutes an antigen-binding site, which is called variable region (V region). Two regions respectively comprising about 400 in heavy chains and 150 amino acid residues in light chains follow the variable region, which regions are constant and common to Ig class and subclass, and called constant region (C region). Human IgG is known to have C$x$ and C$\lambda$ moieties belonging to L chain and C$\gamma$ 1, C$\gamma$ 2, C$\gamma$ 3, and C$\gamma$ 4 moieties belonging to H chain. L, or H chains which are specified by the above moieties are respectively referred to as $x$, $\lambda$, $\gamma$ 1, $\gamma$ 2, $\gamma$ 3, and $\gamma$ 4 chain.

IgM is also a glycoprotein having a molecular weight of about one million and composed of the assembly of five IgG-like constructions. Heavy chain of IgM is referred to as $\mu$ chain (which C region is C$\mu$).

On the other hand, the basic structure of the gene encoding an antibody, i.e., immunoglobulin, is known (See, for instance, S. Tonegawa, Nature, 302 575, 1983). Thus, V region in H chain is coded by V, D, and J genes, while that in L chain is coded by V and J genes. The V, D, and J genes respectively consist of about 300, about 30, and about 50 base pairs. One hundred to two hundred varieties of V genes, four varieties of J genes, and twelve varieties of D genes are involved in the construction of V region of the H chain in mice. One hundred to three hundred varieties of V genes and five varieties of J genes are concerned with the formation of $x$ chain in mice. Through B cells' differentiation, the rearrangement occurs among one of the V genes, one of the D genes, and one of the J genes, and these three selected genes stand in line adjacently to form the V region-encoding gene. The V region-encoding gene in L chain is likewise constructed by the rearrangement of particular V and J genes. A diversity of V region is attributable to a diversity of the combination of particular V, D, and J genes to be selected in H chain, and the combination of particular V and J genes to be selected in L chain. In addition, the rearrangement between V and D, D and J, or V and J genes does not necessarily occur with accurate reproduction and it is often accompanied by shift, deletion, and insertion of some nucleotide(s), which helps increasing the diversity of V region. Furthermore, somatic mutations often occur within the matured V region-encoding gene. which also cause the diversity of the V region.

On the other hand, a series of C region-encoding genes, which determine particular class or sub-class of a given antibody, position downstream of V region-encoding gene. When the rearranged V region-encoding gene is expressed, one of the C region-encoding genes positioned nearest to the V region-encoding gene is co-expressed to yield an antibody polypeptide consisting of V and C regions. Variation of the resulting antibody in the level of "Class", which occurs through B cell's differentiation, could be attributable to the deletion and replacement of the C region-encoding gene positioned nearest to the V region-encoding gene with another C region-encoding gene belonging to another class.

In the present specification, the term, exotoxin of P. aeruginosa is hereinafter referred to as Ex-A. Antibody to Ex-A will be referred to as anti-Ex-A antibody.

V region of an antibody refers to the antigen binding region located at N-terminal of the antibody, which structures is diversified to a large extent. The V region of H chain is encoded by three types of genes, i.e., $V_H$ gene, D gene and $J_H$ gene, while that of $x$ chain is encoded by two types of genes, i.e., $Vx$ gene and $Jx$ gene.

C region of an antibody refers to the region positioned downstream of V region, amino acid sequence of which is common among species of particular class and subclass of antibodies. The gene encoding the C region, which is positioned downstream of J gene, is referred to as $C\mu$ gene, $Cx$ gene, etc, depending on its class and subclass.

$V_H$ gene, D gene, and $J_H$ gene refer to genes, which are parts of V region of the H chain of an antibody. In mice, 100 to 200 $V_H$ genes, 12 D genes, and 4 $J_H$ genes respectively exist in the lump, and each one selected from each lump forms VDJ gene by adjacently standing in line. The VDJ gene corresponds to the V region of the H chain of the antibody.

$Vx$ gene and $Jx$ gene refer to genes, which are part of V region of the $x$ chain of an antibody. In mice, 100 to 300 $Vx$ genes and 5 $Jx$ genes respectively exist in the lump, and each one selected from each lump forms VJ gene by adjacently standing in line. The VJ gene corresponds to the V region of the $x$ chain of the antibody. Thus, V region-encoding gene has the same meaning as VJ gene in L chain and VDJ gene in H chain.

There have already been provided established cell lines producing human monoclonal anti-Ex-A antibody possessing high antibody titer. In addition, it has been found that the monoclonal antibody produced by the above cell lines is effective for the treatment of the infections caused by exotoxin-producing P. aeruginosa (See JP-A-204200/1986). This patent publication discloses that anti-Ex-A antibody-producing cells are human B cell transformed with EB virus, a hybridoma formed by fusing said transformed B cell and mouse myeloma cell, and a hybridoma established by fusing normal human B cell and mouse myeloma cell.

The anti-Ex-A antibody-producing cell lines mentioned above are, of course, very useful as a stable supplier of anti-Ex-A antibody in high concentrations. Unfortunately, however, they are not satisfactory when mass production of the antibody is desired. In mass production, these cells must be modified so that they may be more stable and possess higher productivity. Such modification could be achieved by screening more efficient cell lines through cloning with the aid of "limiting dilution", or by mutating these cells using mutagenic substances. However, these measures are not very efficient, and it appears unlikely to bring any remarkable improvement.

Gene manipulation or genetic engineering has provided a means to produce, in large scale, bioactive substances such as interferons, interleukins, and the like. Gene manipulation is more efficient than the aforementioned. mutagenic procedure in terms of the production of cell lines with high expression efficiency.

On the basis of the above knowledge, the inventors have tried to apply said genetic engineering to the mass production of human anti-Ex-A antibody and succeeded in creating an improved anti-Ex-A antibody-producing cell line. More specifically, the inventors have cloned an gene coding for human anti-Ex-A antibody, and determined the base sequence of the gene corresponding to V region. The inventors have found that human antibody reactive to Ex-A is stably produced with a high productivity by culturing transformant cells which were produced by inserting a human gene encoding the human anti-Ex-A antibody. In particular, the inventors have found that when human cells, especially Namalwa cells are used as the host cells in producing human anti-Ex-A antibody through expression of the human gene encoding the antibody, it can be efficiently produced with the following advantages:

(1) There is no contaminations of the produced antibody with non-human substances which would be produced when non-human host cells are used, and therefore purification can be performed much more easily compared with the cases where non-human host is used.

(2) The produced antibody is accompanied with glycosylation of human cells. When non-human host is used in producing human antibody, the produced antibody contains non-human sugar chains derived from the used host cells, which might cause undesirable immunoreactions of antigenicity to human being. The use of human cells avoids such problems.

Unlike the previous methods which employ a EB virus-transformed human B cell or a human-mouse hybridoma. the present invention provides a very efficient means for the production of human monoclonal anti-Ex-A antibody. which permits more economical and larger scale production of the antibody. For instance, selection of a host cell showing a rapid proliferation leads to the improvement of an economical production of the antibody.

The use of the gene provided by the invention permits a modification of human anti-Ex-A antibody by, for example, substitution of a particular amino acid or acids, deletion or addition of a certain domain, and fusion with other protein. Said modification have been very difficult in previously known methods. The present invention also permits the production of human monoclonal anti-Ex-A antibody substantially free from biohazards by using a host cell which contains no harmful endogenous virus. The present invention thus provides a gene encoding human anti-Ex-A antibody, recombinant plasmids or viruses containing said gene and transformants cells harboring said recombinant plasmids or viruses, in particular human transformant cells capable of producing the anti-Ex-A antibody as the expression product of an inserted gene. It also provides a process for producing the human anti-Ex-A antibody which comprises culturing said transformant cells and recovering the antibody.

More specifically, the present invention provides a gene encoding human anti-Ex-A antibody;

a gene encoding H chain of the anti-Ex-A antibody, which is characterized by the presence of V region-encoding gene accompanied by a signal peptide-encoding gene and an intron, represented by the base sequence (i) listed below;

a gene encoding H chain of the anti-Ex-A antibody, which is characterized by the presence of V region-encoding gene accompanied by a signal peptide-encoding gene represented by the base sequence (ii) listed below;

a gene encoding H chain of the anti-Ex-A antibody, which is characterized by the presence of V region-encoding gene represented by the base sequence (iii) listed below;

a gene encoding L chain of the anti-Ex-A antibody, which is characterized by the presence of V region-encoding gene accompanied by a signal peptide-encoding gene and an intron represented by the base sequence (iv) listed below;

a gene encoding L chain of the anti-Ex-A antibody, which is characterized by the presence of signal peptide-encoding gene represented by the base sequence (v) listed below; and

a gene encoding L chain of the anti-Ex-A antibody, which is characterized by the presence of V region-encoding gene represented by the base sequence (vi) listed below.

(i)

```
         10         20         30         40         50         60
ATGTCTGTCT CCTTCCTCAT CTTCCTGCCC GTGCTGGGCC TCCCATGGGG TCAGTGTCAG
TACAGACAGA GGAAGGAGTA GAAGGACGGG CACGACCCGG AGGGTACCCC AGTCACAGTC

         70         80         90        100        110        120
GGAGATGCCG TATTCACAGC AGCATTCACA GACTGAGGGG TGTTTCACTT TGCTGTTTCC
CCTCTACGGC ATAAGTGTCG TCGTAAGTGT CTGACTCCCC ACAAAGTGAA ACGACAAAGG

        130        140        150        160        170        180
TTTTGTCTCC AGGTGTCCTG TCACAGGTAC AGCTGCAGCA GTCAGGTCCA GGACTGGTGA
AAAACAGAGG TCCACAGGAC AGTGTCCATG TCGACGTCGT CAGTCCAGGT CCTGACCACT

        190        200        210        220        230        240
AGCCCTCGCA GACCCTCTCA CTCACCTGTG CCATCTCCGG GGACAGTGTC TCTAGCAACA
TCGGGAGCGT CTGGGAGAGT GAGTGGACAC GGTAGAGGCC CCTGTCACAG AGATCGTTGT

        250        260        270        280        290        300
GTGTTTCTTG GAACTGGATC AGGCAGTCCC CATCGAGAGG CCTTGAGTGG CTGGGAAGGA
CACAAAGAAC CTTGACCTAG TCCGTCAGGG GTAGCTCTCC GGAACTCACC GACCCTTCCT

        310        320        330        340        350        360
CATACTACAG GTCCAAGTGG TATAATGATT CTGCAGTATC TGTGAAAAGT CGAATAACCA
GTATGATGTC CAGGTTCACC ATATTACTAA GACGTCATAG ACACTTTTCA GCTTATTGGT

        370        380        390        400        410        420
TCAACCCAGA CACATCCAAG AACCAGTTCT CCCTGCAGCT GAACTCTGTG ACTCCCGAGG
AGTTGGGTCT GTGTAGGTTC TTGGTCAAGA GGGACGTCGA CTTGAGACAC TGAGGGCTCC

        430        440        450        460        470        480
ACACGGCTGT GTATTACTGT GTAAGAGCCC GCCCCGGTAC GACCGGGGGT GGTATGGACG
TGTGCCGACA CATAATGACA CATTCTCGGG CGGGGCCATG CTGGCCCCCA CCATACCTGC

        490        500        510
TCTGGGGCCA AGGGACCACG GTCACCGTCT CCTCAG
AGACCCCGGT TCCCTGGTGC CAGTGGCAGA GGAGTC
```

wherein DNA sequences (No. 1-49 and No. 133-143) code for signal peptide and DNA sequence (No. 50-132) codes for intron region.

(ii)

```
         10         20         30         40         50         60
ATGTCTGTCT CCTTCCTCAT CTTCCTGCCC GTGCTGGGCC TCCCATGGGG TGTCCTGTCA
TACAGACAGA GGAAGGAGTA GAAGGACGGG CACGACCCGG AGGGTACCCC ACAGGACAGT

         70         80         90        100        110        120
CAGGTACAGC TGCAGCAGTC AGGTCCAGGA CTGGTGAAGC CCTCGCAGAC CCTCTCACTC
GTCCATGTCG ACGTCGTCAG TCCAGGTCCT GACCACTTCG GGAGCGTCTG GGAGAGTGAG
```

```
              130         140         150         160         170         180
  ACCTGTGCCA TCTCCGGGGA CAGTGTCTCT AGCAACAGTG TTTCTTGGAA CTGGATCAGG
  TGGACACGGT AGAGGCCCCT GTCACAGAGA TCGTTGTCAC AAAGAACCTT GACCTAGTCC

              190         200         210         220         230         240
  CAGTCCCCAT CGAGAGGCCT TGAGTGGCTG GGAAGGACAT ACTACAGGTC CAAGTGGTAT
  GTCAGGGGTA GCTCTCCGGA ACTCACCGAC CCTTCCTGTA TGATGTCCAG GTTCACCATA

              250         260         270         280         290         300
  AATGATTCTG CAGTATCTGT GAAAAGTCGA ATAACCATCA ACCCAGACAC ATCCAAGAAC
  TTACTAAGAC GTCATAGACA CTTTTCAGCT TATTGGTAGT TGGGTCTGTG TAGGTTCTTG

              310         320         330         340         350         360
  CAGTTCTCCC TGCAGCTGAA CTCTGTGACT CCCGAGGACA CGGCTGTGTA TTACTGTGTA
  GTCAAGAGGG ACGTCGACTT GAGACACTGA GGGCTCCTGT GCCGACACAT AATGACACAT

              370         380         390         400         410         420
  AGAGCCCGCC CCGGTACGAC CGGGGGTGGT ATGGACGTCT GGGGCCAAGG GACCACGGTC
  TCTCGGGCGG GGCCATGCTG GCCCCCACCA TACCTGCAGA CCCCGGTTCC CTGGTGCCAG

              430
  ACCGTCTCCT CAG
  TGGCAGAGGA GTC
```

(iii)

```
               IO          20          30          40          50          60
  CAGGTACAGC TGCAGCAGTC AGGTCCAGGA CTGGTGAAGC CCTCGCAGAC CCTCTCACTC
  GTCCATGTCG ACGTCGTCAG TCCAGGTCCT GACCACTTCG GGAGCGTCTG GGAGAGTGAG

               70          80          90         100         110         120
  ACCTGTGCCA TCTCCGGGGA CAGTGTCTCT AGCAACAGTG TTTCTTGGAA CTGGATCAGG
  TGGACACGGT AGAGGCCCCT GTCACAGAGA TCGTTGTCAC AAAGAACCTT GACCTAGTCC

              130         140         150         160         170         180
  CAGTCCCCAT CGAGAGGCCT TGAGTGGCTG GGAAGGACAT ACTACAGGTC CAAGTGGTAT
  GTCAGGGGTA GCTCTCCGGA ACTCACCGAC CCTTCCTGTA TGATGTCCAG GTTCACCATA

              190         200         210         220         230         240
  AATGATTCTG CAGTATCTGT GAAAAGTCGA ATAACCATCA ACCCAGACAC ATCCAAGAAC
  TTACTAAGAC GTCATAGACA CTTTTCAGCT TATTGGTAGT TGGGTCTGTG TAGGTTCTTG

              250         260         270         280         290         300
  CAGTTCTCCC TGCAGCTGAA CTCTGTGACT CCCGAGGACA CGGCTGTGTA TTACTGTGTA
  GTCAAGAGGG ACGTCGACTT GAGACACTGA GGGCTCCTGT GCCGACACAT AATGACACAT

              310         320         330         340         350         360
  AGAGCCCGCC CCGGTACGAC CGGGGGTGGT ATGGACGTCT GGGGCCAAGG GACCACGGTC
  TCTCGGGCGG GGCCATGCTG GCCCCCACCA TACCTGCAGA CCCCGGTTCC CTGGTGCCAG

              370
  ACCGTCTCCT CAG
  TGGCAGAGGA GTC
```

(iv)

```
          10         20         30         40         50         60
ATGGTGTTGC AGACCCAGGT CTTCATTTCT CTGTTGCTCT GGATCTCTGG TGAGGAATTA
TACCACAACG TCTGGGTCCA GAAGTAAAGA GACAACGAGA CCTAGAGACC ACTCCTTAAT

          70         80         90        100        110        120
AAAAGTGCCA CAGTCTTTTC AGAGTAATAT CTGTGTAGAA ATAAAAAAAA TTAAGATATA
TTTTCACGGT GTCAGAAAAG TCTCATTATA GACACATCTT TATTTTTTTT AATTCTATAT

         130        140        150        160        170        180
GTTGGAAATA ATGACTATTT CCAATATGGA TCCAATTATC TGCTGACTTA TAATACTACT
CAACCTTTAT TACTGATAAA GGTTATACCT AGGTTAATAG ACGACTGAAT ATTATGATGA

         190        200        210        220        230        240
AGAAAGCAAA TTTAAATGAC ATATTTCAAT TATATCTGAG ACAGCGTGTA TAAGTTTATG
TCTTTCGTTT AAATTTACTG TATAAAGTTA ATATAGACTC TGTCGCACAT ATTCAAATAC

         250        260        270        280        290        300
TATAATCATT GTCCATTCCT GACTACAGGT GCCTACGGGG ACATCGTGAT GACCCAGTCT
ATATTAGTAA CAGGTAAGGA CTGATGTCCA CGGATGCCCC TGTAGCACTA CTGGGTCAGA

         310        320        330        340        350        360
CCAGACTCCC TGGCTGTGTC TCTGGGCGAG AGGGCCACCA TCAACTGCAA GTCCAGCCAG
GGTCTGAGGG ACCGACACAG AGACCCGCTC TCCCGGTGGT AGTTGACGTT CAGGTCGGTC

         370        380        390        400        410        420
AGTGTTTTAT ACAGCTCCAA CAATAAGAAC TACTTAGCTT GGTACCAGCA GAAACCAGGA
TCACAAAATA TGTCGAGGTT GTTATTCTTG ATGAATCGAA CCATGGTCGT CTTTGGTCCT

         430        440        450        460        470        480
CAGCCTCCTA AGCTGCTCAT TTACTGGGCA TCTACCCGGG AATCCGGGGT CCCTGACCGA
GTCGGAGGAT TCGACGAGTA AATGACCCGT AGATGGGCCC TTAGGCCCCA GGGACTGGCT

         490        500        510        520        530        540
TTCAGTGGCA GCGGGTCTGG GACAGATTTC ACTCTCACCA TCAGCAGCCT GCAGGCTGAA
AAGTCACCGT CGCCCAGACC CTGTCTAAAG TGAGAGTGGT AGTCGTCGGA CGTCCGACTT

         550        560        570        580        590        600
GATGTGGCAG TTTATTACTG TCAGCAATAT TATAGTACTC CTCGTACGTT CGGCCAAGGG
CTACACCGTC AAATAATGAC AGTCGTTATA ATATCATGAG GAGCATGCAA GCCGGTTCCC

         610
ACCAAGGTGG AAATCAAAC
TGGTTCCACC TTTAGTTTG
```

wherein DNA sequence (No. 1-49) and (No. 269-279) code for signal peptide, and DNA sequence (No. 50-268) codes for intron region.

7

(v)

```
         10         20         30         40         50         60
ATGGTGTTGC AGACCCAGGT CTTCATTTCT CTGTTGCTCT GGATCTCTGG TGCCTACGGG
TACCACAACG TCTGGGTCCA GAAGTAAAGA GACAACGAGA CCTAGAGACC ACGGATGCCC

         70         80         90        100        110        120
GACATCGTGA TGACCCAGTC TCCAGACTCC CTGGCTGTGT CTCTGGGCGA GAGGGCCACC
CTGTAGCACT ACTGGGTCAG AGGTCTGAGG GACCGACACA GAGACCCGCT CTCCCGGTGG

        130        140        150        160        170        180
ATCAACTGCA AGTCCAGCCA GAGTGTTTTA TACAGCTCCA ACAATAAGAA CTACTTAGCT
TAGTTGACGT TCAGGTCGGT CTCACAAAAT ATGTCGAGGT TGTTATTCTT GATGAATCGA

        190        200        210        220        230        240
TGGTACCAGC AGAAACCAGG ACAGCCTCCT AAGCTGCTCA TTTACTGGGC ATCTACCCGG
ACCATGGTCG TCTTTGGTCC TGTCGGAGGA TTCGACGAGT AAATGACCCG TAGATGGGCC

        250        260        270        280        290        300
GAATCCGGGG TCCCTGACCG ATTCAGTGGC AGCGGGTCTG GGACAGATTT CACTCTCACC
CTTAGGCCCC AGGGACTGGC TAAGTCACCG TCGCCCAGAC CCTGTCTAAA GTGAGAGTGG

        310        320        330        340        350        360
ATCAGCAGCC TGCAGGCTGA AGATGTGGCA GTTTATTACT GTCAGCAATA TTATAGTACT
TAGTCGTCGG ACGTCCGACT TCTACACCGT CAAATAATGA CAGTCGTTAT AATATCATGA

        370        380        390        400
CCTCGTACGT TCGGCCAAGG GACCAAGGTG GAAATCAAAC
GGAGCATGCA AGCCGGTTCC CTGGTTCCAC CTTTAGTTTG
```

(vi)

```
         10         20         30         40         50         60
GACATCGTGA TGACCCAGTC TCCAGACTCC CTGGCTGTGT CTCTGGGCGA GAGGGCCACC
CTGTAGCACT ACTGGGTCAG AGGTCTGAGG GACCGACACA GAGACCCGCT CTCCCGGTGG

         70         80         90        100        110        120
ATCAACTGCA AGTCCAGCCA GAGTGTTTTA TACAGCTCCA ACAATAAGAA CTACTTAGCT
TAGTTGACGT TCAGGTCGGT CTCACAAAAT ATGTCGAGGT TGTTATTCTT GATGAATCGA

        130        140        150        160        170        180
TGGTACCAGC AGAAACCAGG ACAGCCTCCT AAGCTGCTCA TTTACTGGGC ATCTACCCGG
ACCATGGTCG TCTTTGGTCC TGTCGGAGGA TTCGACGAGT AAATGACCCG TAGATGGGCC

        190        200        210        220        230        240
GAATCCGGGG TCCCTGACCG ATTCAGTGGC AGCGGGTCTG GGACAGATTT CACTCTCACC
CTTAGGCCCC AGGGACTGGC TAAGTCACCG TCGCCCAGAC CCTGTCTAAA GTGAGAGTGG

        250        260        270        280        290        300
ATCAGCAGCC TGCAGGCTGA AGATGTGGCA GTTTATTACT GTCAGCAATA TTATAGTACT
TAGTCGTCGG ACGTCCGACT TCTACACCGT CAAATAATGA CAGTCGTTAT AATATCATGA

        310        320        330        340
CCTCGTACGT TCGGCCAAGG GACCAAGGTG GAAATCAAAC C
GGAGCATGCA AGCCGGTTCC CTGGTTCCAC CTTTAGTTTG G
```

The invention also provides a gene encoding the H chain of human Ex-A antibody, which is characterized in that it contains the base sequence encoding the V region represented by the amino acid sequence (vii) or (viii); and a gene encoding the L chain of human Ex-A antibody, which is characterized in that it contains the base sequence encoding the V region represented by the amino acid sequence (ix) or (x).

(vii)MetSerValSerPheLeuIlePheLeuProValLeuGlyLeuProTrpGlyValLeuSer GlnValGlnLeuGlnGlnSer-GlyProGlyLeuValLysProSerGlnThrLeuSerLeu

ThrCysAlaIleSerGlyAspSerValSerSerAsnSerValSerTrpAsnTrpIleArg GlnSerProSerArgGlyLeuGluTr-pLeuGlyArgThrTyrTyrArgSerLysTrpTyr AsnAspSerAlaValSerValLysSerArgIleThrIleAsnProAspThrSerLysAsn GlnPheSerLeuGlnLeuAsnSerValThrProGluAspThrAlaValTyrTyrCysVal ArgAlaArgProGlyThrThr-GlyGlyGlyMetAspValTrpGlyGlnGlyThrThrVal ThrValSerSer

wherein amino acid sequence from Met to Ser (No. 1-20) show sigunal peptide.

(viii)GlnValGlnLeuGlnGlnSerGlyProGlyLeuValLysProSerGlnThrLeuSerLeu ThrCysAlaIleSerGlyAspSer-ValSerSerAsnSerValSerTrpAsnTrpIleArg

GlnSerProSerArgGlyLeuGluTrpLeuGlyArgThrTyrTyrArgSerLysTrpTyr AsnAspSerAlaValSerValLysSerAr-gIleThrIleAsnProAspThrSerLysAsn GlnPheSerLeuGlnLeuAsnSerValThrProGluAspThrAlaValTyrTyrCysVal Ar-gAlaArgProGlyThrThrGlyGlyGlyMetAspValTrpGlyGlnGlyThrThrVal ThrValSerSer

(ix)MetValLeuGlnThrGlnValPheIleSerLeuLeuLeuTrpIleSerGlyAlaTyrGly AspIleValMetThrGlnSer-ProAspSerLeuAlaValSerLeuGlyGluArgAlaThr

IleAsnCysLysSerSerGlnSerValLeuTyrSerSerAsnAsnLysAsnTyrLeuAla TrpTyrGlnGlnLysProGlyGln-ProProLysLeuLeuIleTyrTrpAlaSerThrArg

GluSerGlyValProAspArgPheSerGlySerGlySerGlyThrAspPheThrLeuThr IleSerSerLeuGlnAlaGluAspValAlaVal-TyrTyrCysGlnGlnTyrTyrSerThr ProArgThrPheGlyGlnGlyThrLysValGluIleLys

wherein amino acid sequence from Met to Gly (No. 1-20) show signal peptide.

(x)AspIleValMetThrGlnSerProAspSerLeuAlaValSerLeuGlyGluArgAlaThr IleAsnCysLysSerSerGlnSer-ValLeuTyrSerSerAsnAsnLysAsnTyrLeuAla

TrpTyrGlnGlnLysProGlyGlnProProLysLeuLeuIleTyrTrpAlaSerThrArg GluSerGlyValProAspArgPheSerGlySer-GlySerGlyThrAspPheThrLeuThr IleSerSerLeuGlnAlaGluAspValAlaValTyrTyrCysGlnGlnTyrTyrSerThr ProArg-ThrPheGlyGlnGlyThrLysValGluIleLys

The present invention also provides recombinant plasmids or viruses containing said genes and recombinant <u>Escherichia</u> <u>coli</u>, mouse myeloma cell or human cell harboring said recombinant plasmids or viruses.

The present invention further provides

H chain of human anti-Ex-A antibody, which is characterized by the presence of V region accompanied by a signal peptide represented by the amino acid sequence (vii),

H chain of human Ex-A antibody, which is characterized by the presence of V region represented by the amino acid sequence (viii),

L chain of human Ex-A antibody, which is characterized by the presence of V region accompanied by a signal peptide represented by the amino acid sequence (ix),

L chain of human Ex-A antibody, which is characterized by the presence of V region represented by the amino acid sequence (x), and

Human anti-Ex-A antibody harboring the above L chain and/or H chain.

The present invention further provides a process for the production of a human monoclonal antibody to Ex-A, which process comprises culturing the transformant cells harboring the recombinant plasmids or viruses containing said genes and recovering human monoclonal anti-Ex-A antibody.

Especially, human cell, such as Namalwa cell, is desirous as a host cell in the above method.

A gene encoding the human anti-Ex-A antibody, which may be either a genomic DNA or cDNA, can be obtained from cell lines producing human monoclonal antibody against exotoxin of <u>P</u>. <u>aeruginosa</u>. Such cell lines can be obtained by the methods disclosed in

JP-A-204200/1986. The genes encoding the H chains and L-chains, the V regions of which are represented by the DNA sequences (i) through (vi) or by the amino acid sequences (vii) though (x) can be isolated from the human monoclonal anti-Ex-A antibody-producing cell line, FK-001 disclosed in the Japanese patent publication (Kokai) No. 204200/1986.

The genomic DNA encoding anti-Ex-A antibody can be obtained by the following gene cloning procedure:

9

(i) Isolation of genomic DNA from human anti-Ex-A antibody-producing cell

The isolation of the genomic DNA from said cells consists of dissolving or lysing the cells with proteinase K in the presence of SDS, removing proteins and RNA by phenol extraction and ribonuclease treatment respectively, and extracting and purifying the DNA (see, for instance, MENEKI JIKKEN SOSA HO Vol 12, 3981-3986, 1983, JAPAN MENEKI GAKKAI).

(ii) Preparation of gene library of anti-Ex-A antibody-encoding cell

A gene library can be prepared by complete or partial digestion of the genomic DNA obtained above by the use of restriction enzymes or physical treatment such as ultrasonication, followed by the insertion of the resulting DNA fragments into a phage vector. The insertion of the fragments can be conducted by the methods known in the art filed of molecular biology. For this cloning purpose, various phage vectors can be employed, such as EMBL3 (A. Frischauf et al., J. Mol. Biol., 170 827-842, 1983), λ charon 4A (F. Flatter et al., Science, 196 161, 1977), and λ gtWES λB' (P. Ledere et al., Science, 196 175, 1977), which are commercially available. EMBL3 is provided by Promega Biotech., and λ charon 4A and λ gtWES λB' are available from Amersham.

(iii) Screening and analysis of desired gene encoding anti-Ex-A antibody

The gene encoding anti-Ex-A antibody is obtained by screening the library obtained above using various probes. For the screening of the gene encoding the H chain of the antibody, a DNA fragment coding for $J_H$ gene (J.V. Ravetch et al., Cell, 27 583-591, 1981) may be employed as a probe. In the case where the H chain is μ chain, a DNA fragment coding for Cμ gene is preferably employed (N. Takahashi et al., Nucleic Acids Res. 8 5983, 1980). The screening of the genes for x chain and λ chain of the L chain may be conducted by the use of, as a probe, Jx gene (P.A. Hieter et al., J. Biol. Chem., 257 1516-1522, 1982), and C λ gene (P.A. Hieter et al., Nature, 294 536, 1981), respectively. These probes are available from Japanese Cancer Research Resources Bank (JCRB).

Plaques containing the desired gene can be selected by known method, such as plaque hybridization (W.D. Benton & R.W. Davis, Science, 196 180, 1977), using the probes labelled with $^{32}$P by nick translation (P.W.J. Rigby et al., J. Mol. Biol., 113 237, 1977).

Phage DNA is then extracted and purified from the selected phage plaques containing the antibody encoding gene in a conventional manner (T. Maniatis et al., "Molecular Cloning" p85, 1972). A gene map may be prepared on the basis of restriction analysis of the DNA and Southern hybridization with the aforementioned probes (E. Southern, J. Mol. Biol., 98 503, 1975). The base sequence of the desired region of the map may be determined by dideoxy chain termination method (J. Messing, "Methods in Enzymol" vol. 101, 20-78, 1983) and Maxam Gilbert method (A.M. Maxam & W. Gilbert, Proc. Natl. Acad. Sci. U.S.A., 74 560, 1977). In the case where the base sequence contains sense codons, the corresponding amino acid sequence may be estimated.

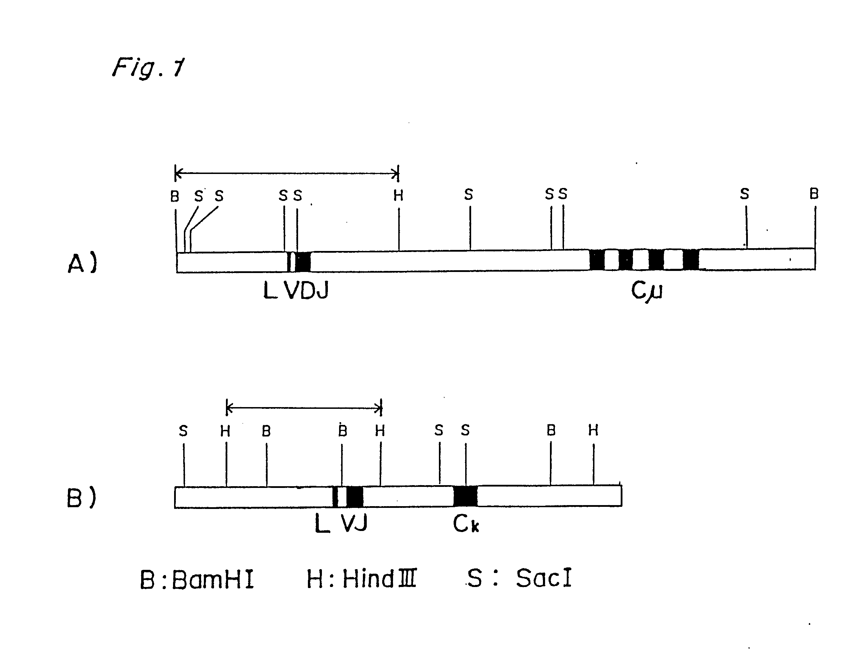

λ gFK1 is a typical example of phage clones which contain the antibody-encoding gene obtainable by the above procedures. λ gFK1 is composed of phage vector EMBL3 and 11.5 kb (kiro base) of Sau3A-partially digested DNA fragment encoding the V and C regions of x chain inserted into Bam HI site of EMBL3. Another example is λ gFM12 which consists of phage vector EMBL 3 and ~15 kb BamHI-completely digested DNA fragment encoding the V and C regions of μ chain, which has been inserted into the BamHI site of the vector. The detailed structures of these antibody-encoding genes cloned by EMBL3 are shown in Fig. 1 of the accompanying drawings.

cDNA which encodes anti-Ex-A antibody may be prepared using a conventional cloning technique as outlined below.

(i) Isolation of mRNA from human anti-Ex-A antibody-producing cell

The isolation of the mRNA from said cells consist of dissolving or lysing the cells in the presence of a protein denaturing agent (e.g. guanidine thiocyanate), isolating mRNA by phenol extraction or cesium chloride density-gradient ultracentrifugation, and purifying the mRNA using oligo(dT)cellulose column (see, for example, "Molecular Cloning", p187, 1982).

(ii) Preparation of cDNA library

The preparation of cDNA library can be conducted in accordance with known methods, such as Gubular-Hoffmann method (a kit for the method is available from Amersham), Okayama-Berg method (Mol. Cell. Biol., 2 161-170, 1982), Land method, Gubular-Hoffmann-linker method (a kit for the method is available from Amersham), etc. In the Example described below, Okayama-Berg method gives a cDNA library comprising 100,000-400,000 clones using 5-30 µg of RNA and 1.4 µg of primer DNA, and a cDNA library comprising 2,000,000 clones starting from 1-2 µg of mRNA and 1.4 µg of primer DNA.

(iii) Screening and analysis of human anti-Ex-A antibody-encoding gene

The cDNA encoding anti-Ex-A antibody is obtainable by screening the library obtained above by the use of various probes. The probes can be prepared by restriction digestion of a human gene encoding antibody available from JCRB. The probes are also obtained in the following manner.

A consensus sequences in $J_H$ and $J_L$ genes of mouse antibody can be obtained from a gene data base (EMBL). Based on this information, complementary oligo DNAs of the following sequences are synthesized:

JL:    5'-TGAT($\begin{smallmatrix}T\\C\end{smallmatrix}$)TCCACCTTGG-3'

JH-1:    5'-AGGAGAC($\begin{smallmatrix}G\\T\end{smallmatrix}$)GTGA($\begin{smallmatrix}C\\G\end{smallmatrix}$)($\begin{smallmatrix}C\\A\end{smallmatrix}$)G-3'
$\phantom{xxxxxxxx}\overset{T}{\phantom{x}}$

JH-2:    5'-T($\begin{smallmatrix}G\\C\end{smallmatrix}$)CCTTG($\begin{smallmatrix}A\\G\end{smallmatrix}$)CCCCAGT-3'

The above synthetic DNAs are used as a probe for screening human anti-Ex-A antibody-encoding gene. For instance, the JL probe listed above is employed for the cloning of the gene encoding human x chain from cDNA library of lymphoid cell, GM1 500 (HGMCR). The resulting cDNA for human x chain is useful as a probe for screening the x chain-encoding gene from cDNA library or genomic library of anti-Ex-A antibody producing cell, such as FK-001 by colony-hybridization ("Molecular Cloning", p309, 1982) or another screening method.

The gene encoding human anti-Ex-A antibody obtained as above can be integrated into a suitable expression vector by conventional methods, and the vector is, in turn, used for transformation of adequate host cells for production of human monoclonal anti-Ex-A antibody. Construction of expression vector containing the gene and transformation of host cells with the expression vector can be carried out, for example, in the following procedures:

Construction of expression vector containing the genomic DNA

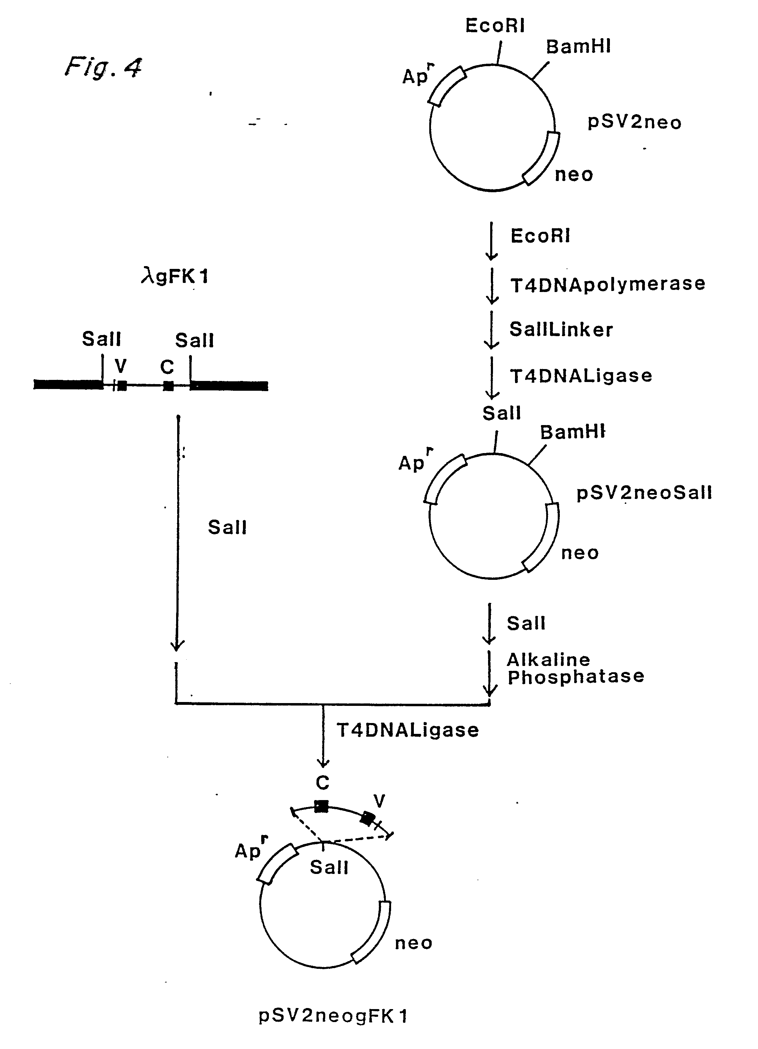

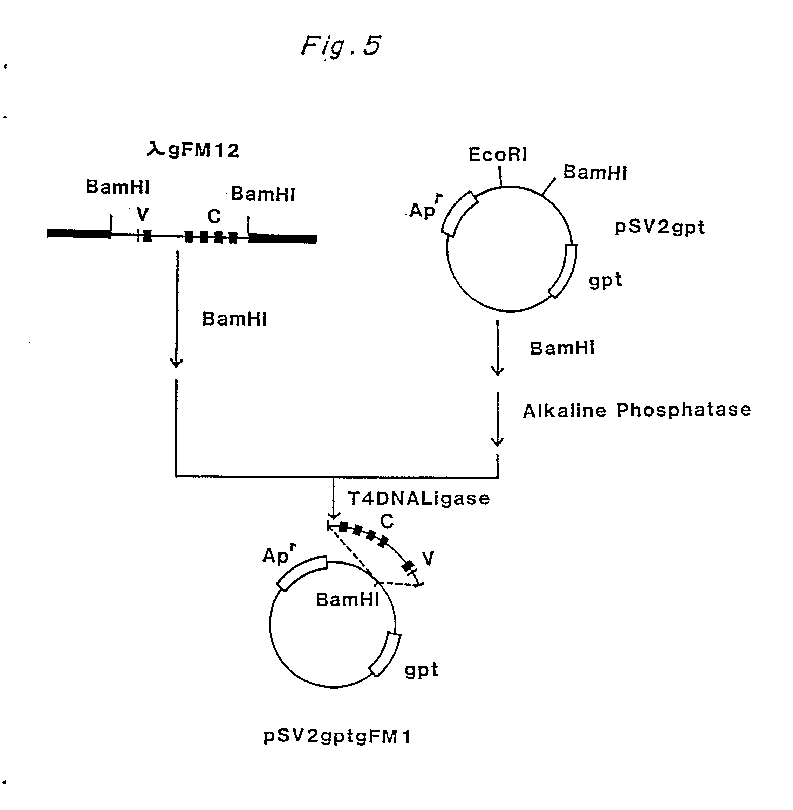

The anti-Ex-A antibody-encoding genomic DNA cloned in phage vector is cleaved from the vector by digesting with an adequate restriction enzymes and inserted into a vector functional in animal host cells. Specific examples of such vectors for animal cells are pSV2gpt (R.C. Mulligan & P. Berg, Science, 209 1422, 1980), pSV2neo (P.J. Southern & P. Berg, J. Mol. Appl. Genet., 1 327, 1982), BPV vector (N. Saver et al., Mol. Cell. Biol., 1 486, 1981), an adenovirus vector (N. Jones & T. Shenk, Cell, 13 181, 1978), and a retrovirus vector (C. Cepke et al., Cell, 37 1053, 1984). The examples using pSV2gpt and pSV2neo are illustrated in Example 1-(6) hereinafter described and also in Figs. 4 and 5. The pSV2gpt and pSV2neo are available from Bethesda Research, and the other vectors are obtainable from JCRB.

For construction of the expression vector, expression of the gene, transformation of Escherichia coli, extraction and purification of plasmid DNA can be carried out by conventional procedures.

E. coli DH1 (B. Low, Proc. Natl. Acad. Sci. U.S.A. 60 160, 1968, JCRB), E. coli JM83 (C. Yanisch-Perron et al., Gene, 33 103, 1985, JCRB), and other E. coli K-12 strains are employable. (Bachmann, Bacteriol. Rev., 86 525-557, 1972). These microorganisms are available from American Type Culture Collection. A wide variety of bacterium other than E. coli can also be used known in the art.

Transformation can be conducted in conventional manner (for instance, S.N. Cohen et al., Proc. Natl. Acad. Sci., U.S.A. 69 2110, 1972, and T. Maniatis et al., "Molecular Cloning" 249-253, 1982).

The extraction and purification of plasmid DNA can be conducted by known methods, such as alkaline denaturation, cleared lysate, and combination thereof with cesium chloride gradient centrifugation (T. Maniatis et al., "Molecular Cloning" 1982).

Transfection of animal cell with expression vector

Transfection of animal cells, such as a cell line derived from human, monkey. chimpanzee, baboon, hamster, rat, or mouse cell, with the expression vector harboring the desired gene can be conducted in accordance with known methods such as calcium phosphate technique (M. Wigler et al., Cell, 11 223, 1977), protoplast fusion technique (W. Schaffner, Proc. Natl. Acad. Sci. U.S.A., 77 2163, 1980), and electroporation technique (H. Potter et al., Proc. Natl. Acad. Sci. U.S.A., 81 7161-7165, 1984). Human cell. such as Namalwa cell, is desirous as a host cell. For instance, pSV2gptgFM1. pSV2neogFK1 or pSV2dhfr (S. Subramani et al., Mol. Cell. Biol., 1 854, 1981), which has been linearized with an appropriate restriction enzyme, is introduced into mouse myeloma cell by electroporation technique. The resultant transformant is cultured and selected in a medium containing agent G418 or a mixture of mycophenolic acid and hypoxanthine to establish a cell line.

Expression of anti-Ex-A antibody-encoding gene

The drug-resistant cells obtained above are cultured, and colonies capable of expressing the desired gene are selected by measuring the content of anti-Ex-A antibody in the culture medium. The L chain and H chain of the antibody can be separately measured by enzyme immunoassay, particularly by ELISA. Antigen-binding activity can also be determined by using an antigen plate. Expression of anti-Ex-A antibody-encoding gene can also be detected by other known methods such as RIA, Western blotting method, Northern Blotting method, etc.

Construction of expression plasmid containing cDNA encoding the human anti-Ex-A antibody-encoding gene

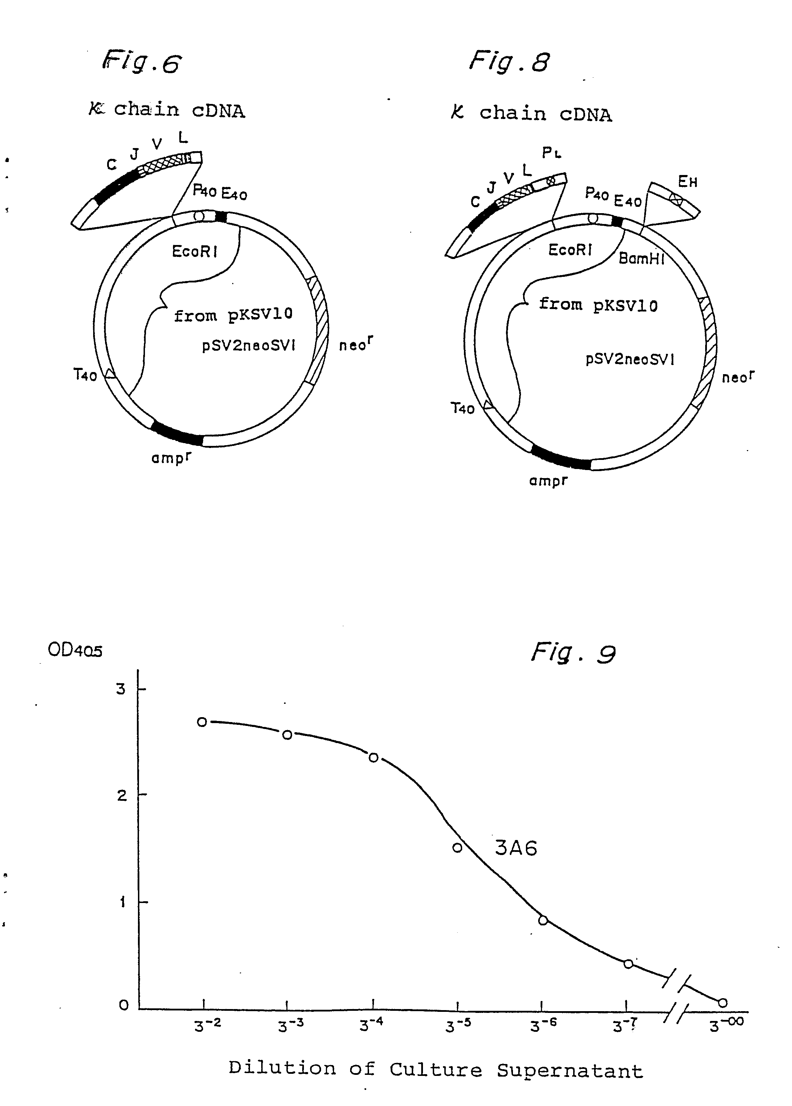

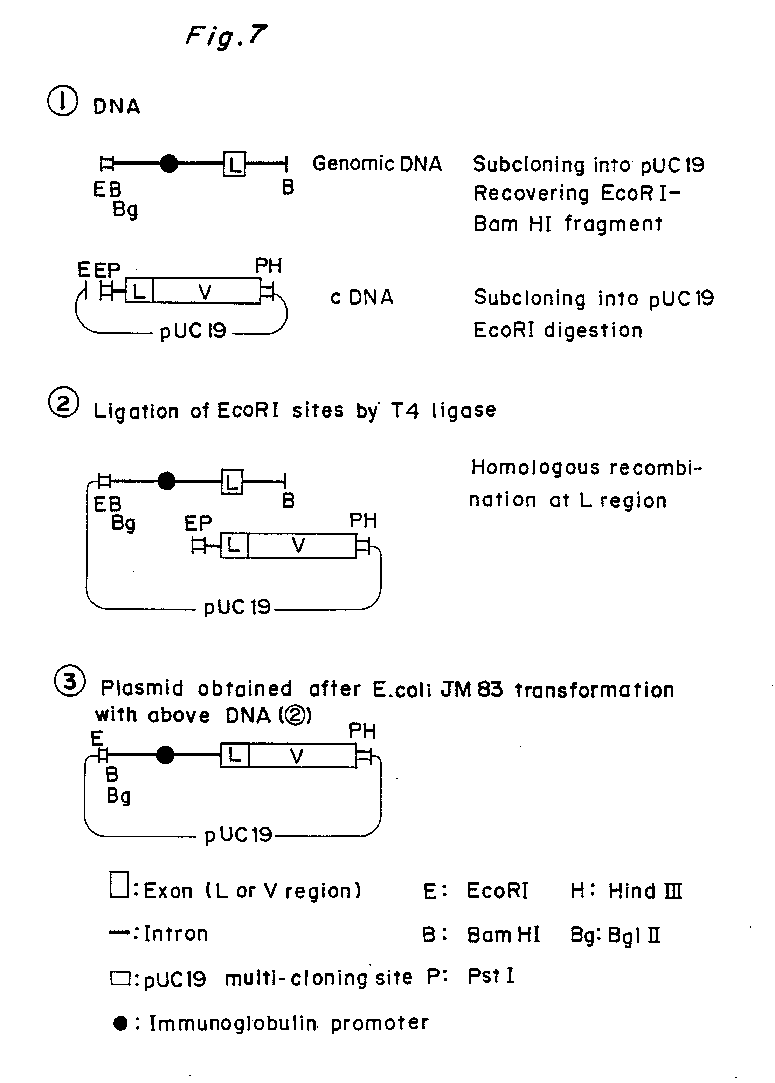

Essentially, the construction of an expression plasmid for the cDNA gene can be conducted in the similar manner to the aforementioned procedure for the genomic gene. Appropriate promoter and terminator must be linked to the cDNA gene during the construction. Particularly, SV40 promoter is linked to 5′ end of the cDNA, and SV40 terminator to 3′ end, which enables the expression of the cDNA in animal cells. If desired, the promoter associated with the gene encoding Ex-A antibody and an appropriate enhancer may be additionally incorporated into the plasmid for the purpose of regulating the expression. Examples 3-(4) and 3-(5) illustrate the use of pSV2neo as a starting plasmid (see Figs. 6 and 7).

Transfection of animal cell with expression vector

Transfection can be conducted in the same manner as described before in connection with genomic gene. Example 2-(6) describes the transfection using protoplast fusion technique. Only a kind of DNA can incorporate into the host cell when protoplast fusion technique using PEG1500 (45% PEG, 10% DMSO) or PEG4000 is employed. Accordingly, it is preferred to employ two step procedure, in which x chain is first incorporated into a host cell and μ chain is then incorporated into the transformed host cell which has been confirmed to exhibit high degree of expression with respect to the x chain. In this manner, the cell line which produces human monoclonal anti-Ex-A antibody can be established.

Expression of anti-Ex-A antibody-encoding gene and isolation and purification of it are conducted as described before.

Expression of the anti-Ex-A antibody may be conducted in genus of Escherichia, Bacillus, or Saccharomyces as well as animal cells by the use of a suitable vector. Specific example of such vector for use in Escherichia coli is pKK223-3 (Pharmacia). In fact, it is clear that cloned cDNA encoding IgM antibody is expressive in E. coli or yeast in connection with appropriate promoter and ribosome binding site (M.A. Boss et al. Nucleid Acids Research, 12 (1984) 3791; C.R. Wood et al., Nature, 314 (1985) 446). As mentioned previously, human cells, especially Namalwa cells are preferable for the production of the human monoclonal anti-Ex-antibody in accordance with the method of the present invention.

The following detailed examples are presented by way of illustration of certain specific embodiments of the invention. The restriction enzymes and linkers available from Takara Syuzo Inc. were employed unless otherwise stated.

In the accompanying drawings:

Fig. 1 is restriction site and functional map of the gene encoding anti-Ex-A antibody isolated from FK-001. A) and B) respectively represent μ chain from λ gFM12 and x chain of the gene from λ gFK1.

Fig. 2 is base sequence of Vx region of FK-001 derived from λ gFK1. (No. 532-882 DNA sequence encodes V region.)

Fig. 3 is base sequence of $V_H$ region of FK-001 derived from λ gFM12. (No. 317-689 DNA sequence encodes V region.)

Fig. 4 is construction of plasmid pSV2neogFK1.

Fig. 5 is construction of plasmid pSV2gptgFM1.

Fig. 6 is a restriction site and functional map of plasmid pSV2neoSV1FK1-1.

Fig. 7 is construction of gc-chimeric gene for x chain.

Fig. 8 is a restriction site and functional map of plasmid pSV2neoSV1gcFK1.

Fig. 9 shows a binding activity of anti-Ex-A antibody to Ex-A produced by the clone 3A6 determined by ELISA using Ex-A plate.

In the drawings, following abbreviations and symbols are employed.

$E_{40}$ .....SV40 enhancer

$P_{40}$ .....SV40 promoter

EH......enhancer for H chain of the antibody

PL......promoter for x chain of the antibody

$T_{40}$ .....$SV_{40}$ terminator

## Example 1

Cloning of genomic DNA encoding anti-Ex-A antibody, determination of the base sequences of V regions, and expression of the antibody in mouse myeloma cell

### (1) Isolation of human genomic DNA

Human Ex-A antibody-producing cells FK001($10^8$ cells) were ruptured by grinding with a glass rod and suspended in a buffer (10 mM Tris-HCl, pH 8.0/ 10 mM EDTA/10 mM NaCl). After the addition of proteinase K(Boehringer Mannheim Inc.) and SDS to a concentration of 200 μg/ml and 0.5 %, respectively, the resultant suspension was incubated at 37°C for 90 minutes. To the suspension was added an equal volume of water-saturated phenol, and the mixture was stirred and centrifuged to separate the aqueous layer from the phenol layer. The aqueous layer was recovered, and two volumes of cold ethanol was added thereto. The aqueous solution was gently stirred with a glass rod to precipitate DNA, which was collected by winding up with the rod. After drying in vacuo, the DNA was suspended in 3 ml of TE buffer (10 mM Tris-HCl, pH 8.0/1 mM EDTA), and ribonuclease A (Sigma) was added to the suspension to a concentration of 50 μg/ml, and the mixture was incubated at 37°C for 30 minutes. After the addition of an equal volume of solution of phenol and chloroform (1:1), the mixture was stirred and centrifuged to separate the layers. The aqueous layer was recovered and 2 volumes of cold water were added to precipitate DNA, which was then recovered by winding up with a glass rod as described above. After drying in vacuo, the DNA was suspended in 2 ml of TE buffer. This yielded about 600 μg of human genomic DNA.

### (2) Preparation of library of human genomic gene

To each of three portions (each 20 μg) of the genomic DNA (prepared in Example 1-(1)) was added 1.7 units of restriction enzyme Sau3AI in TA buffer (33 mM Tris-acetic acid, pH 7.9/66 mM potassium acetate/10 mM magnesium/0.5 mM DTT) and each of the resultant mixtures was incubated at 37°C for 10, 20, and 30 minutes, respectively, and then at 65°C for 5 minutes. After the incubation, these reaction mixtures were combined to obtain a partially digested product. On the other hand, 10 μg of the genomic DNA prepared in Example 1-(1) was incubated with 150 units of restriction enzyme Bam HI at 37°C for 2 hours and the reaction was terminated by heating at 65°C for 5 minutes to obtain the completely digested product. These digestion products were incubated with 2 units of calf-intestinal alkaline phosphatase in 10 mM Tris-HCl, pH 8.0/0.1 mM EDTA at 37°C for 30 minutes, and the DNA was recovered by phenol extraction and ethanol precipitation. The DNA was subsequently ligated to the BamHI site of linear phage vector EMBL3 DNA (Promega Biotec.) which had been prepared by digesting intact EMBL3 DNA (50μg) with 100 units of each of restriction enzymes BamHI and EcoRI in a TA buffer at 37°C for 2 hours and inactivating the enzymes by heating at 65°C for 5 minutes. Ligation was carried out by reacting 24 μg of the vector DNA with 3 μg of the digested genomic DNA, and 500 units of T4 DNA ligase in 100 μl of 66 mM Tris-HCl, pH 7.6/6.6 mM MgCl$_2$ /10 mM DTT/1 mM ATP at 16°C for 16 hours. The resulting DNA was subjected to in vitro packaging (A. Becker and M. Gold, Proc. Natl. Acad. Sci., USA, 72 581, 1975) to obtain a human gene library (3 × 10$^7$ p.f.u./100μl).

(3) Screening of gene encoding human anti-Ex-A antibody

From the library of human gene prepared in Example 1-(2), 5 × 10$^5$ plaques were prepared using E. coli LE 392 (JCRB) as a bacterial indicator. The phages present in these plaques were transferred onto a nitrocelullose filter by the replica method and screened for the gene encoding human anti-Ex-A antibody in accordance with the plaque hybridization method (W.D. Benton and R.W. Daris, Science, 196 180.1977). In this method, human antibody J$_H$ gene and Cμ gene (JCRB) were used as a probe for the screening of μ chain gene, and human antibody Jx gene (P. A. Hieter et al. J. Biol. Chem. 257 (1982) 1516) and Cx gene (cloned from GM1500 as described in example 2-(3)) were used as a probe for the screening of x chain gene. These probes were previously labeled with $^{32}$P by nick translation method (a kit was obtained from Amersham Inc.) before the plaque hybridization. 31 phage clones hybridizing with human antibody J$_H$ probe were isolated from the library (5 × 10$^5$ phages) prepared from the BamHI digested genomic DNA, and 5 phage clones hybridizing with human antibody Jx probe were isolated from the library (1 × 10$^6$ phages) prepared from the Sau3A digested genomic DNA.

(4) Analysis of phage clone harboring antibody-encoding gene

Phage lysate was prepared from the phage clone obtained in Example 1-(3), and phage DNA was extracted from the lysate (see T. Maniatis, E.F. Fritsh, and J. Sambrook, "Molecular Cloning", 1982, Cold Spring Harbor Laboratory). The phage DNA was digested with restriction enzyme (5 units/0.5μg of phage DNA) in TA buffer at 37°C for 2 hours and the digestion products of said DNA was fractionated by 0.8-2.4 % agarose gel electrophoresis. DNA was stained by soaking the gel in a solution of ethidium bromide (1 μg/ml) and the fluorescence generated by the formation of DNA-ethidium bromide complex was observed by exposing to UV light. The size of the digestion products (kilo base pairs, kb) was determined by comparison of the mobility of the digested products with that of the co-electorophorated HindIII-digested λ DNA (Pharmacia).

In the same manner as above, the restriction enzyme cleavage sites were determined and "restriction enzyme cleavage map" of the phage DNA was prepared. The electrophoretically fractionated restriction fragments were transferred onto a nitrocellulose filter, and the V and C regions of the resultant phage DNA were identified by means of the southern hybridization analysis (E. Southern, J. Mol. Biol., 98, 503; 1975) using $^{32}$P-labeled J$_H$ or Jx genes and $^{32}$P-labeled Cμ or Cx genes as a probe respectively.

(5) Determination of the base sequence of the gene encoding V region and its flanking region

Determination of the base sequence of the gene which encodes the V region of x chain and μ chain was conducted as follows. The 3.5 kb HindIII restriction fragment containing V region of x chain and the 5.2 kb BamHI-HindIII restriction fragment containing V region of μ chain (corresponding to fragments marked with arrows in Fig. 1 (B), (A) respecrively) were subcloned respectively into the HindIII and BamHI-HindIII sites of the cloning vector pUC18 (C. Yanisch-Perron, J. Vieira, and J. Messing, Gene, 33, 103, 1985: Pharmacia, Inc.). Namely, restriction enzyme HindIII (10 units) and a combination of restriction enzymes HindIII and BamHI (each 10 units) were respectively added to 2 μg of the phage λ gFK1 DNA containing x chain gene and 2 μg of the phage λ gFM1 DNA containing μ chain gene, and each of the mixtures was incubated in 20 μl of TA buffer at 37°C for 2 hours, followed by the incubation at 65°C for 5 minutes for inactivation of the enzymes. The DNA was collected by ethanol precipitation and suspended in 10 μl of TE buffer. pUC18 DNA (each 0.5 μg) was digested with 5 units of HindIII, or 5 units of each of HindIII and BamHI, under the same conditions as above, and the resultant DNA fragments were collected from each reaction mixture by ethanol precipitation. The resultant DNA fragments were reacted with 0.2 unit of calf-intestinal alkaline phosphatase under the same conditions described in Example 1-(2), and the DNA fragments were collected by phenol extraction and ethanol precipitation and suspended in 5 μl of TE buffer.

Each of the digestion products (1 μg) of x chain gene or μ chain gene was incubated with the corresponding digested vector (0.2 μg) in 20 μl of a reaction buffer (see, Example 1-(2)) containing 100 units of T4 DNA ligase at 16°C for 16 hours.

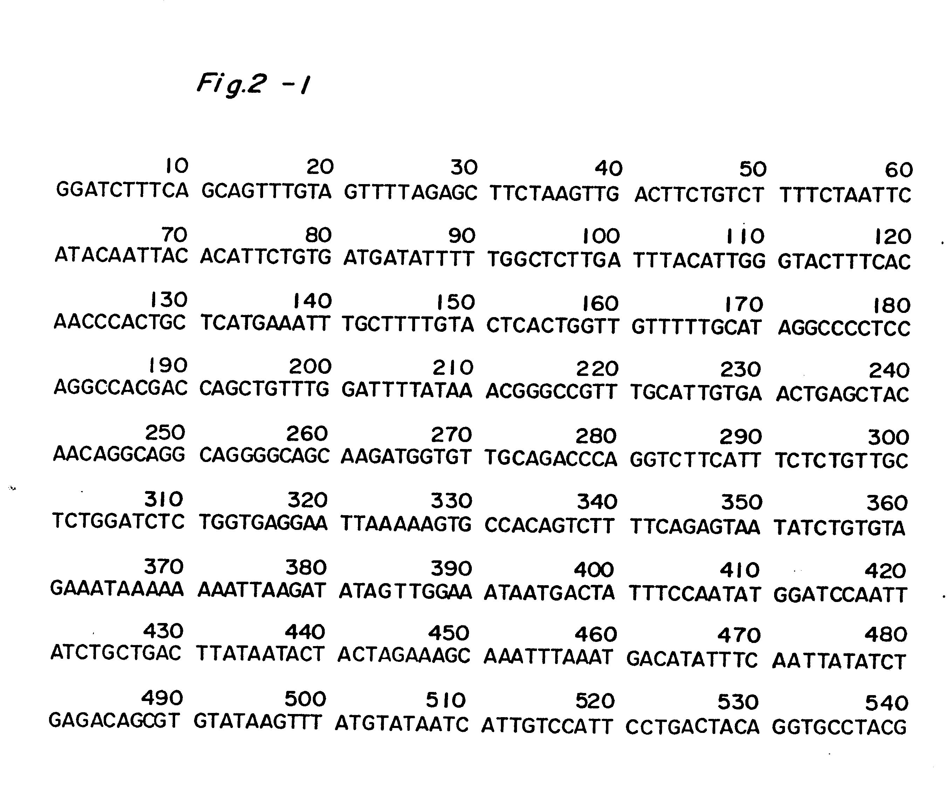

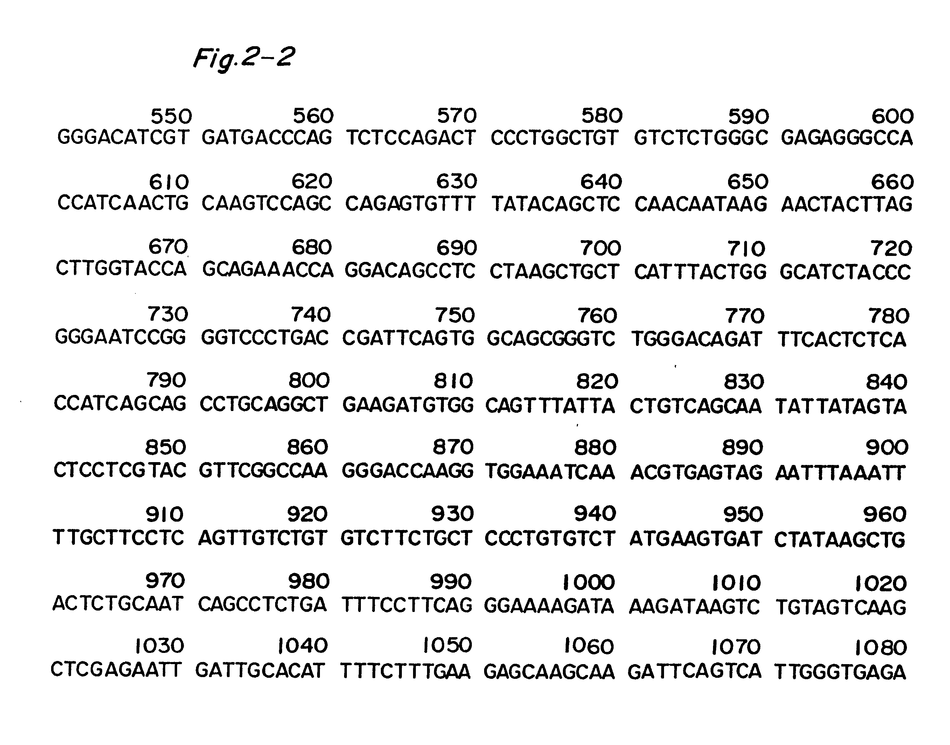

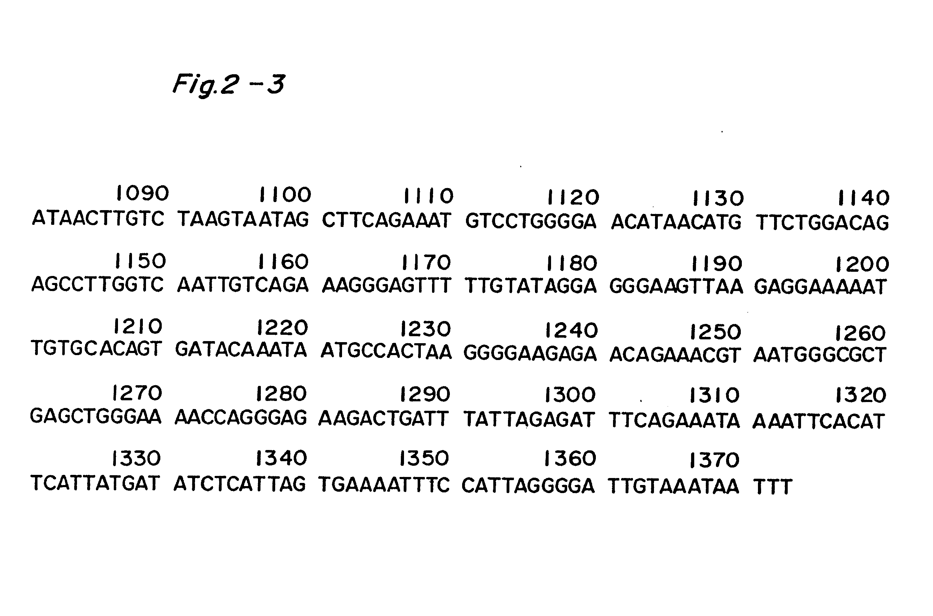

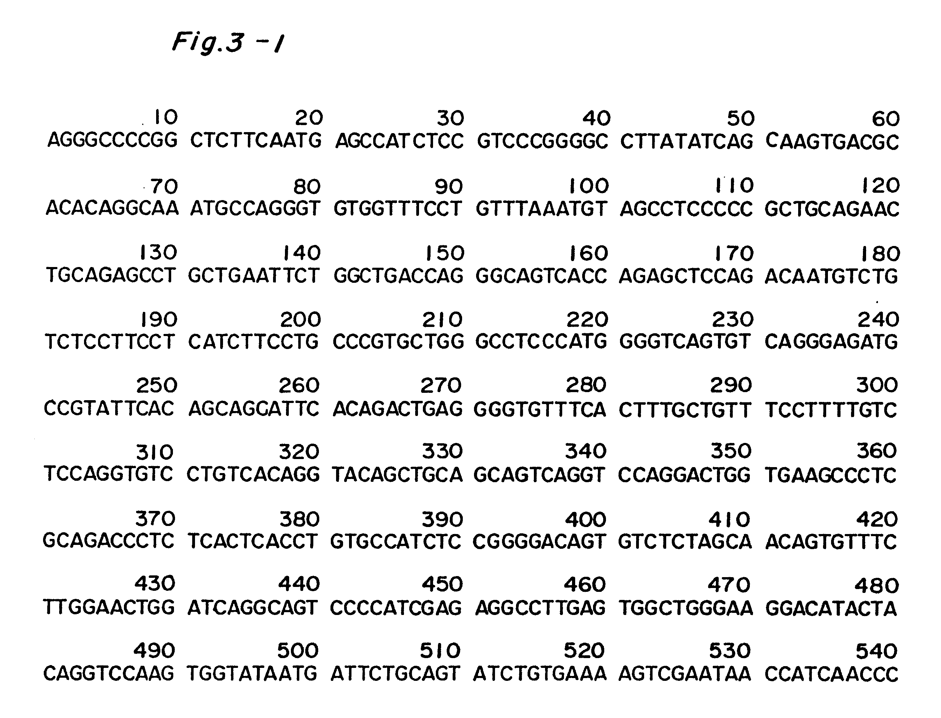

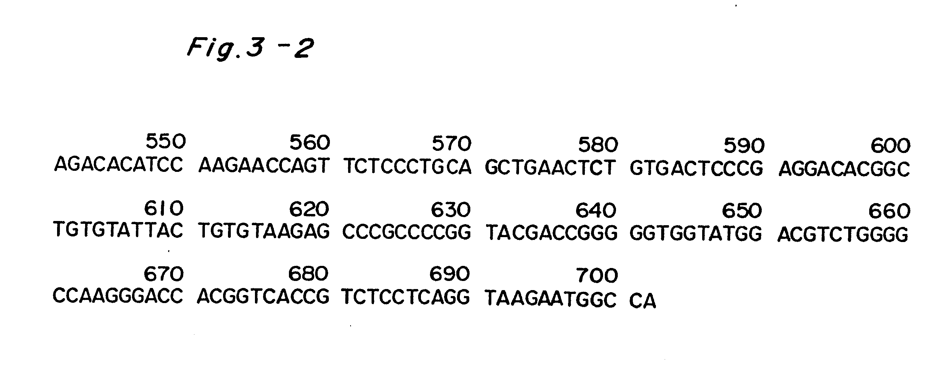

The ligation mixture was used to transform E. coli JM83 (C. Yanisch - Perron. J. Vieira, and J. Messing, Gene, 33, 103, 1985; JCRB). White colonies resistant to 50 μg/ml ampicillin were selected in the presence of 2 mM isopropyl thiogalactoside and 40 μg/ml of X-gal, and cultivated. Plasmid DNA was extracted from the culture using alkaline lysis method and digested with the restriction enzyme HindIII. or restriction enzymes HindIII and BamHI, and electrophoresed on agarose gel (refer to Example 1-(4)). After staining the gel with ethidium bromide, plasmids pgFKV carrying VJ gene of x chain and pgFM6 carrying VDJ gene of μ chain were selected. Various restriction enzyme cleavage sites present in these plasmids were identified, and the restriction enzyme maps were prepared. Various deletion derivatives of V region-encoding gene were prepared using restriction sites of the gene in the following manner. Various types of deletions which expand from the multicloning site of vector pUC18-encoding region to the V region-encoding gene was introduced by cleaving the plasmid with various kinds of restriction enzymes followed by re-ligation with T4 DNA ligase. When the enzyme that cleaved the V region-encoding gene was not identical to the enzyme which was employed for cleaving the multicloning site, their cohesive ends were converted to blunt ends using T4 DNA polymerase before the re-ligation procedure. The resultant re-ligation mixture was used to transform E. coli DH1 and the ampicillin (50 μg/ml)-resistant strains were selected. Plasmid DNAs were extracted and their restriction patterns were investigated for the purpose of the selection of the desired deletion derivatives. The resulting deletion derivatives were denatured with 0.2N NaOH, and the DNAs were recovered by ethanol precipitation. The base sequences of the DNAs of various parts of the V region gene were determined by chain terminator method using dideoxynucleotides (13 Sequencing Kit available from Takara Syuzo Inc. and the accompanying protocol were employed). The information of these partial base sequences were combined and the entire base sequence encoding both V region and its flanking region was determined (Figs. 2 and 3) (J. Messing "Methods in Enzymology" 101 (1983) 20-78). Precise location of V region gene of FK-001 was determined by comparing amino acid sequences deduced from its base sequences with them so far identified (E. A. Kabat et al. "Sequences of Proteins of Immunological Interest (1983) MIH). Restriction maps of FK-001 μ chain and x chain gene including V and C regions are illustrated in Fig. 1.

(6) Construction of expression plasmid

Five μg of vector pSV2neo (P.J. Southern and P. Berg, J. Mol. Appl. Genet., 1, 327, 1982: Bethesda Research Laboratory) was incubated in 20 μl of TA buffer containing 20 units of restriction enzyme EcoRl at 37°C for 2 hours, and EcoRl restriction fragment was collected by ethanol precipitation. The DNA fragment (2 μg) was incubated with T4 DNA polymerase (1 unit) in 30 μl of a reaction buffer containing 67 mM Tris-HCl, 6.7 mM MgCl$_2$ , 10 mM 2-mercaptoethanol, 6.7 uM EDTA, 16.6 mM (NH$_4$)$_2$SO$_4$ . each 330 μM of dCTP, dATP, dGTP and dTTP, at 37°C for 30 minutes to convert the cohesive ends to blunt ends, and then the DNA fragment was collected by phenol extraction and ethanol precipitation. The DNA fragment (1 μg) was incubated with 0.1 μg of Sall linker in the presence of 100 units of T4 DNA ligase in 20 μl of a reaction buffer (see, Example 1-(2)) at 16°C for 16 hours, and the ligation mixture was used to transform E. coli. DH1. Plasmid DNA was extracted from the resulting ampicillin resistant cells and the desired plasmid, which has Sall restriction site instead of EcoRl restriction site, was selected by investigating the Sall restriction pattern. The obtained plasmid was designated pSV2neoSall. Each 1 μg of plasmid pSV2neoSall and λ gFK1 were separately incubated with 5 units of restriction enzyme Sall in 20 μl of a reaction buffer (TA buffer containing 150 mM NaCl) at 37°C for 2 hours and the DNAs were recovered by phenol extraction and ethanol precipitation. Sall-digested plasmid pSV2neoSall (1 μg) was treated with 0.5 unit of calf-intestinal alkaline phosphatase (as Example 1-(5)) and the DNA was recovered by phenol extraction and ethanol precipitation. The DNA was then mixed with 1 μg of Sall-digested λ gFK1 and the mixture was incubated in 20 μl of a reaction solution in the presence of 100 units of T4 DNA ligase at 16°C for 16 hours (as done in Example 1-(2)). The resultant ligation mixture was used to transform E. coli DH1 and the plasmid DNA was extracted from the ampicillin resistant colony. Desired ligation product of x chain-encoding gene and pSV2neo was selected by the restriction analysis and designated pSV2neogFK1 (Fig. 4) (FERM BP-1548). Each 1 μg of vector pSV2gpt (R.C. Mulligum and P. Berg, Science, 209, 1422. 1980; Bethesda Research Laboratory) and λ gFM12 were separately incubated with 15 units of restriction enzyme BamHI in 20 μl of TA buffer at 37°C for 2 hours and the digested DNAs were recovered by phenol extraction and ethanol precipitation. BamHI digestion product (1 μg) of the plasmid pSV2gpt was treated with 0.5 unit of calf-intestinal alkaline phospnatase (as done in Example 1-(5)) and the DNA was recovered by phenol extraction and ethanol precipitation. The resulting DNA was then mixed with 1 μg of BamHI digestion products of λ gFM12, and the mixture was treated in accordance with the procedure described for the preparation of the pSV2gFK1. This yielded plasmid pSV2gptgFM1 (FERM BP-1550) containing the μ chain gene ligated to the plasmid pSV2gpt (Fig. 5).

(7) Transfection of mouse myeloma cells with the expression plasmids and espression of the antibody

Each 40 μg of pSVneo2gFK1, pSV2gptgFM1, and pSV2dhfr (S. Subramani et al., Mol., Cell, Biol., 1. 854, 1981; Bethesda Research Laboratory) was incubated with 100 units of restriction enzyme PvuI in 200 μl of a reaction buffer (TA buffer/150 mM KCl) at 37°C for 6 hours and then at 65°C for 5 minutes, and the DNA was collected by ethanol precipitation. The DNA was suspended in 100 μl of a modified PBS (0.58 g/l NaCl, 1.6 g/l KCl, 1.15 g/l $Na_2 HPO_4$ , 0.2 g/l $KH_2 PO_4$ , 5 mM $MgSO_4$ ) and the suspension was incubated at 65°C for 20 minutes. Mouse myeloma cells P3 x 63-Ag8.653 (ATCC) ($10^7$ cells) which had been grown in RPM11640-10% FCS to a density of 5 x $10^5$ cells/ml, were washed by centrifugation in a modified PBS and suspended in 400 μl of the modified PBS. To the cell suspension was added 100 μl of the above-mentioned DNA suspension, and the mixture was transferred into a plastic cell equipped with the electrodes of thin aluminum film at both sides. The distance between two electrodes were about 3 mm. After allowing to stand for 10 minutes in an ice bath, the cell was exposed to a high voltage pulse (6 xVcm$^{-1}$) using Power Supply (Model 494; ISCO Co. Ltd.,) and allowed to stand for another 10 minutes in ice. To the cell-DNA suspension was added 10 ml of RPMI1640 medium, and the mixture was incubated at 37°C for 2 hours in the presence of 5% $CO_2$ and centrifuged. The resultant cell were suspended in 20 ml of RPMI1640-10% FCS and the suspension was pipetted onto two 96-well plates. After 3 days incubation of plates in the presence of 5% $CO_2$ at 37°C, antibiotic G418 (Gibco Inc.) was added to a concentration of 400 μg/ml and the incubation was continued while exchanging the half volume of the medium every 3 or 4 days. G418 resistant colonies were observed after 10 day incubation, which were tested for the expression of human anti-Ex-A antibody.

(8) Detection of human anti-Ex-A antibody

Anti-ex-A antibody activity was measured as follows. Each of Ex-A, sheep antibody to human x chain (Cappel) and rabbit antibody to human μ chain (Cappel) was dissolved in PBS(-) (8 g/l NaCl, 0.2 g/l KCl. 2.9 g/l $Na_2 HPO_4$ .$12H_2 O$, 0.2 g $KH_2 PO_4$ ) to a concentration of 0.5-1.0 μg/ml and the solution was pipetted into a 96-well plate (Falcon) (120 μl/well) previously washed with water ( x 3), and the plate was incubated at 37°C for 2 hours. After the plate was dried in vacuo 1% bovine serum albumin (BSA) added PBS(-) was pipetted into each well (200 μl/well) and the incubation was continued at 37°C for 2 hours. After removal of water, the plate was dried in vacuo. The cultured supernatant of G418 resistant colony, which obtained in Example 1-(7) was appropriately diluted with 1% BSA added PBST[0.05% Tween 20 (Wako Junyaku) added PBS(-)], and the diluted solution was pipetted into the above-mentioned plates (100 μl/well), which were then incubated at 37°C for 2 hours. After removal of solution and washing out of the supernatant with PBST from each plate, alkaline phosphatase (ALP)-labeled goat antibody to human μ chain (Tago)(100 μl/well) was pipetted into the aforementioned Ex-A plate and the plate containing rabbit antibody to human μ chain, while ALP-labeled antibody to human x chain (Tago)(100 μl/well) was pipetted into the plate containing goat antibody to human x chain, and these plates were incubated at 37°C for 2 hours, dewatered and washed in PBST. To each plate was added 10% diethanolamine buffer (pH 9.1) containing 1 mg/ml of p-nitrophenylphosphate (100 μl/well) and the plate was incubated at 37°C for 15 minutes. The amounts of the human Ex-A antibody, μ chain and x chain thereof were assayed by measuring the $OD_{405}$ using a multiscanner. The test results are presented in the following Table.

16

## Table

| Clone | OD$_{405}$ | | |
| | Ex-A antibody[1] | μ chain[2] | κ chain[2] |
|---|---|---|---|
| 1A2 | 0.369 | 1.771 | 2.134 |
| 1A3 | 0.131 | 0.247 | 0.709 |
| 1A10 | 0.689 | 2.210 | 2.519 |
| 1A11 | 0.019 | 0.081 | 0.531 |
| 1C3 | 0.395 | 1.249 | 1.429 |
| 1C8 | 0.011 | 0.060 | 0.659 |
| 1D3 | 0.348 | 1.808 | 2.071 |
| 1D7 | 0.003 | 0.085 | 0.585 |
| 1E4 | 0.697 | 1.508 | 2.380 |
| 1E10 | 0.034 | 0.082 | 0.442 |
| 1F3 | 0.611 | 2.161 | 2.474 |
| 1F9 | 0.050 | 0.297 | 0.573 |
| 1F10 | 0.008 | 0.109 | 0.509 |
| control | 0.057 | 0.096 | 0.063 |

\* 1) original solution    2) 9 fold dilution

The table shows that the clones 1A10, 1E4, and 1F3 possess high anti-Ex-A antibody titer. The amount of the antibody produced by 1A10 was 34 μg/ml, which was greater than that of the EB virus-transformed cell line FK-001. Clones 1C3, 1D3, 1A2, and 1A3 were also observed to express antibodies having activity of binding to Ex-A. It can be concluded from these results that the H and L chains of anti-Ex-A antibody were simultaneously expressed, and assembled to form the stereochemical structure capable of binding to Ex-A.

## Example 2

### Production of human anti-Ex-A antibody with human cells

(1) Transfection of human B cell line with the expression plasmids and expression of the antibody

Each 80 μg of pSV2neogFK1 and pSV2gptgFM1 was incubated with 200 units of restriction enzyme Pvu I in 300 μl of a reaction buffer (TA buffer/150 mM KCl) at 37°C for 3 hours and then at 65°C for 5 minutes, and the DNA was collected by ethanol precipitation. The DNA was suspended in 120 μl of PBS(-)

(8 g/l NaCl, 0.2 g/l KCl, 2.9 g/l $Na_2$ $HPO_4$ • $12H_2$ O, 0.2 g/l $KH_2$ $PO_4$ ) and the suspension was incubated at 65°C for 20 minutes. Namalwa cell (ATCC CRL-1432, human B cell line) ($2.2 \times 10^7$ cells) which had been cultured in RPM11640-10% FCS added with Penicillin G (500 units/ml) and sulfate streptomycine (100 mg/ml) were washed by centrifugation in PBS(-) and suspended in 0.9 ml of the PBS(-). To the cell suspension was added the abovementioned DNA suspension, and the mixture was transferred into a plastic cell equipped with the electrodes of thin aluminum film at both sides. The distance between two electrodes were about 6 mm. After allowing to stand for 10 minutes in an ice bath, the cell was exposed to a high voltage pulse ($3 \ xVcm^{-1}$) using Power Supply (Model 494: ISCO Co. Ltd.,) and allowed to stand for another 10 minutes in ice. To the cell-DNA suspension was added 10 ml of RPMI1640 medium, and the mixture was incubated at 37°C for 2 hours in the presence of 5% $CO_2$ and centrifuged. The resultant cell were suspended in RPMI1640-10% FCS ($3 \times 10^5$ cells/ml) and the suspension was pipetted onto 96-well plates (0.1 ml/well). After 3 days incubation of plates in the presence of 5% $CO_2$ at 37°C. Same amounts of RPMI1640-10%FCS medium containing antibiotic G418 (Gibco Inc.) (400 mg/ml or 800 $\mu$g/ml) was added to the plates and the incubation was continued while exchanging the half volume of the medium (containing G418 (400 $\mu$g/ml)) every 3 or 4 days. G418 resistant colonies were observed after 11 day incubation, which were tested for the expression of human Ex-A antibody.

(2) Detection of human anti-Ex-A antibody

Anti-Ex-A antibody activity was measured as follows. Each of Ex-A (Switzerland seahrum and vaccine institute) and goat antibody to human $\mu$ chain (Cappel) was dissolved in PBS(-) and the solution was pipetted into a 96-well plate (Falcon) (100$\mu$l/well) previously washed with distilled water, and the plate was incubated at 37°C for 2 hours. After removal of solution, the plate was dried in vacuo to prepare the assay plates. The cultured supernatant of G418 resistant colony, which obtained in Example 2-(2) was appropriately diluted with PBST [PBS(-) added with 0.05% Tween 20 (Wako Junyaku)] added PBS(-)], and the diluted solution was pipetted into the above-mentioned plates (100 $\mu$l/well), which were then incubated at 37°C for 2 hours. After removal of solution and washing out of the supernatant with PBST from each plate, alkaline phosphatase (ALP)-labeled goat antibody to human $\mu$ chain (Tago, diluted to 1/500 with PBST)(100 $\mu$l/well) was pipetted into the aforementioned Ex-A plate and the plate containing goat antibody to human $\mu$ chain, and these plates were incubated at 37°C for 2 hours, dewatered and washed in PBST. To each plate was added 10% diethanolamine buffer (pH 9.1) containing 1 mg/ml of p-nitrophenylphosphate (100 $\mu$l/well) and the plate was incubated at 37°C for 15 minutes. The amounts of the human anti-Ex-A antibody, $\mu$ chain thereof were assayed by measuring the $OD_{405}$ using a multiscanner(FLOW). The test results are presented in the following Table.

18

### Table

| Clone | OD$_{405}$ | |
|---|---|---|
| | Ex-A antibody[1] | μ chain |
| 1C6 | 0.225 | 0.504 |
| 1H11 | 0.213 | 0.707 |
| 2H5 | 0.173 | 0.430 |
| FK-001 | 0.233 | – |
| Namalwa | 0.050 | 0.051 |
| IgM[2] | – | 0.988 |

* 1) 5 fold dilution of culture supernatant with PBST

   2) purified IgM (0.5μg/ml) (Milds)

The table shows that the clones 1C6, 1H11, and 2H5 possess anti-Ex-A antibody titer and production of human μ chain. It can be concluded from these results that anti-Ex-A antibody, which is almost same as that produced by FK-001, is produced in Namalwa cell by introducing the anti-Ex-A antibody encoding gene of FK-001 into it.

The highest possible cell densities of the above clones are higher than that of FK-001 (obtain clones: $3 \sim 5 \times 10^6$ cells/ml, FK-001: $<10^6$ /ml) and their growing rates are twice as rapid as that of FK-001, which show that these clones are superior to FK-001 in cell growth. The rate of introduction of DNA to Namalwa cell is about 10 times as high as that to mouse myeloma cell, so that Namalwa cell is superior as a host cell to produce antibody.

(3) The detection of anti-Ex-A antibody produced by the transformed Namalwa cell with Western blotting method

Supernatant of culture of clone 1H11 (Example 2-(2)) (50 μl) was subjected to 12.5% polyakryl amido gel electrophoresis (SDS-PAGE) in the presence of dodecyl sodium sulfate in accordance with Laemmli method (Nature, 227 (1970) 680).

The resultant gel was soaked in the buffer (25 mM Tris/192mM glycin (pH 8.3)/20% methanol) to equilibrate, on which nitrocellurose film was placed. The protein on the gel was transferred to the nitrocellurose film by exposing it to 10V electricity for 16 hours in the same buffer, while the gel is faced to cathode.

The resultant nitrocellurose film was immersed in a buffer (50 mM Tris (pH 8.0)/0.9% NaCl) (TBS) containing 20% BSA at room temperature for 60 minutes, and allowed to react in TBS buffer containing 0.2 BSA and 1/200 volume peroxydase-labeled sheep-antibody to human μ chain (Bio-Yada) or peroxydase-labeled goat antibody to human x chain (Cappel) for 2~3 hours.

After the above nitrocellurose film was washed with TBS several times, it was stained with TBS containing 0.5 mg/ml 3,3'-diaminobentizine (DAB) and 0.05% $H_2$ $O_2$ .

Human μ chain and x chain are specifically stained with peroxydase labeled antibody to human μ chain and that to human x chain, respectively (Western Blott method). Clone 1H11 was confirmed to produce human μ chain with a molecular weight of 84,000 and human x chain with a molecular weight of 27,000.

When the supernatant of the clone was subjected to electrophoresis on 4~20% gradient gel without reduction procedure and to Western Blott method using antibody to human x chain. a band was found at the position of about 1,000,000 of molecular weight. This results suggest that the anti-Ex-A antibody derived from clone 1H11 is IgM-specific pentameric structure.

(4) Purification of the antibody produced by the Namalwa cell and detection of contaminant protein by Western Blott Method

Clone 1H11 was cultivated in a serum-free medium, Celgrosser H (Sumitomo Pharmaceutical Co. Ltd.), using ACUSYST P (Endotronics). 0.5 l of culture supernatant obtained above was subjected to column chromatography (pharmacia Q sepharose Fast Frow, Fharmacia). After adsorption with 0.15 M phosphate buffer (pH 6.5) and elution with 0.5 M phosphate buffer, it was confirmed that almost cell of IgM was eluted by ELISA. The fraction was concentrated 10 fold by ultrafiltration using YM-10 (Amicon) (Protein concentration is about 11 mg/ml). The resultant fraction (5 ml) was subjected to gel filtration using Sephacryl S-300 column (bed volume is almost 400 ml, pharmacia) with PBS(-). IgM was found in the first protein fraction. A fraction containing the IgM derived from clone IF3 (Example 1-(8)) was obtained in the same manner as above. The IgM fractions obtained above were subjected to SDS-PAGE and resultant gel was silver-stained (silver-stained kit was prepared by DAIITI KYAKU). Clear bands, which correspond to μ chain or x chain, and several unclear bands due to a small amount of contaminants were observed on the gel. The contaminants were identified by Western Blott Method as follows. The antibody used in the method was rabbit antiserum prepared from rabbits immunized with sepernatant of mouse myeloma cell 653, which was obtained by sonification of the cell ($5 \times 10^8$ cells) and centrifugation (2000 x g, 30 minutes).

The rabbit anti-mouse cell antiserum was diluted 200 fold with TBS containing 0.2% BSA and used for Western Blotting. After the nitrocellulose filter was washed with TBS, protein derived from the mouse myeloma cell 653 was stained using peroxydase labeled goat antibody to rabbit IgG.

The contaminant protein in IgM fraction derived from clone IF3 was stained and that derived from clone 1H11 was not stained. Consequently there was no contamination with the protein derived from cells of other kinds of animals in IgM fraction of clone 1H11, i.e., the contamination contained in the above fraction is derived from human cell, except bovine insulin which is contained in the medium Celgrosser H.

When bovine insulin is substituted with human insulin, the anti-Ex-A antibody produced by the transformed human cell line 1H11 will contain no non-human protein. This will eliminate a danger of anaphylaxie shock which might be induced by administration of non-human impurities.

Example 3

Cloning of cDNA encoding anti-Ex-A antibody, determination of its base sequence, and expression of the antibody encoded by the cDNA

(1) Isolation of mRNA from human anti-Ex-A antibody-producing cell

Human monoclonal anti-Ex-A antibody-producing cell, FK-001 ($1.6 \times 10^8$) was suspended in 16 ml of a 4M guanidine thiocyanate solution at 60°C. After reducing the viscosity of the resultant suspension by passing it through a 18G injection needle, 16 ml of phenol was added thereto at 60°C, and the mixture was shaken vigorously. To the mixture were added 8 ml of 0.1 M sodium acetate (pH 5.2), 10 mM Tris buffer (pH 7.4), 1 mM EDTA, and 16 ml of a mixture of chloroform and isoamyl alcohol(24:1), and the resultant mixture was vigorously shaken while maintaining the temperature at 60°C. After cooling to 0°C in an ice bath, the mixture was centrifuged to separate the aqueous layer, from which RNA (1 mg) was isolated by ethanol precipitation.

(2) Preparation of cDNA library for human anti-Ex-A antibody

The preparation of the library was conducted in substantial accordance with the teaching of Okayama-Berg method. Primer DNA (purchased from Pharmacia Inc.)(1.4 μg) and the RNA (30 μg) obtained above were mixed together in a reaction mix (50 mM Tris, pH 8.3, 10 mM $MgCl_2$ , 80 mM KCl, 5 mM DTT, 2 mM dATP, 2 mM dCTP, 2 mM dGTP, 2 mM dTTP) containing reverse transcriptase (purchased from

20

Seikagaku Kogyo) t:êe the complementary DNA. After addition of dC to the complementary strand. the DNA was circularized by the use of a linker DNA (purchased from Pharmacia Inc.), and repaired by the use of T4 DNA ligase, polymerase I (purchased from Takara Syuzo), and RNaseH (purchased from Wako Junyaku). The resultant DNA was used to transform $\underline{E.\ coli}$ DH1, which gave a cDNA library consisting of $4 \times 10^5$ clones.

(3) <u>Screening and analysis of cDNA encoding human anti-Ex-A antibody</u>

A consensus sequence of mouse $J_L$ gene shown below was synthesized and used as a $J_L$ probe:

$$\text{TGAT}(\genfrac{}{}{0pt}{}{T}{C})\text{TCCACCTTGG}$$

The probe was used to detect the clones harboring x chain-encoding cDNA from the cDNA library of human x chain-producing cell GM1500 (HGMCR, Human Genetic Mutant Cell Repository, U.S.A.) by colony hybrydization (M.

Grunstein and Hegness, Proc. Natl. Acad. Sci., 72, 396, 1975). FK-001 cDNA library was then screened using the x chain-encoding cDNA as a probe to obtain cDNA encoding x chain of FK-001. Restriction enzyme map and determination of the base sequence of the cDNA coding for the antibody was accomplished in the same manner as in Example 1-(4) and 1-(5). The test results agreed with those obtained from genomic DNA (Fig. 1) (corresponding to base sequence of No. 237-312 and No. 532-882 in Fig. 2). The screened clone of FK-001 cDNA was named as pcFKl.

(4) <u>Construction of expression plasmid for human anti-Ex-A antibody (i)</u>

The plasmid capable of expressing the cDNA which encodes human anti-Ex-A antibody was constructed in the same manner as in Example 1-(6). After converting the <u>Bg</u>lII site of pKSV-10 (Pharmacia Inc.) to <u>Eco</u>RI site, <u>Kpn</u>I-<u>Bam</u>HI restriction fragment was cleaved. The <u>Kpn</u>I-<u>Bam</u>HI restriction fragment, which contains the regions coding for SV40 promoter, enhancer and terminator, was inserted into the EcoRI site of the pSV2neo (Bethesda Research Laboratory) to construct the expression plasmid for the cDNA gene. The plasmid was designated as pSV2neoSV1. On the other hand, x chain cDNA was subcloned in pUC18 and pUC19 (Pharmacia Inc.), and <u>Eco</u>RI site was generated at both terminals of the cDNA. The <u>Eco</u>RI restriction fragment of x chain cDNA was inserted into the <u>Eco</u>RI site of the above-mentioned plasmid pSV2neoSV1 to form an expression plasmid for x chain (designated as pSV2neoSV1FK1-1 (FERM BP-1549)) (Fig. 6).

(5) <u>Construction of expression vector for human anti-Ex-A antibody (ii)</u>

Construction of the plasmid was carried out in such a way described in example 1-(6). The x chain genomic DNA of FK-001 has the promoter region upstream of leader region. We adopted <u>in vivo</u> homogeneous recombination procedure (for instance Nogami et. al. J. Bacteriol. 164 797-801, 1983) to construct the DNA composing Ig promoter region and x chain cDNA. The steps for construction of the plasmid are shown in Fig. 7.

The BglII-HindIII fragment (1370bp) of genomic DNA derived from λ gFK-1, which includes L, V and J region of x chain gene was cloned by vector pUC 19. EcoRI-BamHI fragment (430 bp) containing the promoter and L region was extracted from the resultant plasmid by low-melting-point agarose gel electrophoresis.

On the other hand, the PstI-PstI fragment containing cDNA of L, V region of x chain, derived from pcFK1 was subcloned into the PstI site of plasmid pUC19, and the resultant plasmid was digested with EcoRI.

The EcoRI fragment and EcoRI-BamHI fragment containing x chain genomic DNA prepared above were mixed and ligated by T4 DNA ligase. The linear DNA as shown Fig. 7-(2) was obtained. $\underline{E.\ coli}$ JM83 were transformed with the above linear DNA by standard $CaCl_2$ methods (Molecular Cloning, Cold Spring Harbor, 1982). Transformants containing circular plasmids formed by homologous recombination within L region were obtained. From this plasmid, EcoRI-PstI fragment containing genomic DNA encoding Ig promoter and cDNA encoding Ig L and V region was isolated by preparative low-melting-point agarose gel electrophoresis.

On the other hand, the human Ig enhancer DNA (JCRB, Japanese Cancer Research Resources Bank. Japan) was inserted into the unique BamHI site of vector pSV2neoSV1 (the above-mentioned). and the resultant plasmid was termed pSV2neoSV1E5.

PstI-PvuII fragment specifying $C_x$ region of FK-001 derived from pcFK1 was subcloned into pUC18. From this plasmid, PstI-EcoRI fragment corresponding to the above PstI-PvuII fragment was isolated.

Then the two types of EcoRI-PstI fragments prepared above, one of which contains genomic DNA encoding Ig promoter and cDNA encoding L and V region, the other of which contains cDNA encoding $C_x$ region were inserted into the unique EcoRI site of pSV2neoSV1E5 in tandem. Consequently, the expression plasmid pSV2neoSV1gcFK1 (Fig. 8) which had the genomic DNA encoding Ig promoter and cDNA encoding L, V and $C_x$ region was constructed.

(6) <u>Transfection of animal cell with the expression vector and expression of the antibody</u>

Protoplasts of E. coli. DH1 harboring aforementioned pSV2neoSV1gcFK1-1 or pSV2neoSV1gcFk1 were prepared in 4 ml of a solution of 10% sucrose, 0.5 mg/ml lysozyme (sigma), and 50 mM EDTA, and diluted with a mixture of 15 ml of 10% sucrose and 10 mM $MgCl_2$. The protoplast ($4.7 \times 10^8$) was mixed with mouse myeloma Sp2/0 ($1.3 \times 10^7$ cells) which had been grown in RPMI1640-10% FCS to a density of $6 \times 10^5$ cell/ml, and a fusion reaction was carried out using PEG4000 (BDH. 47.5% PEG). When the fusion was completed, the mixture was suspended in 65 ml of RPMI1640-10% FCS, and the suspension was spread on a 24-well plate (0.5 ml/well) and incubated at 37°C under 5% $CO_2$. After addition of 0.5 ml of RPMI1640-10% FCS containing 400 μg/ml G418 (Gibco) on 1st day, and then 1 ml on 3rd day, the incubation was continued while exchanging part of the medium (1 ml each) with the same fresh medium every 2 or 3 days. G-148 resistant colonies were observed on 16th day.

(7) <u>Detection of human anti-Ex-A antibody</u>

Rabbit antibody to human x chain (Cappel) was placed on a plate in accordance with the same procedure as of Example 1-(8). To the plate was pipetted (100 μl/well) the supernatant of previously described culture (Example 3-(6)) containing drug resistant strains, and the plate was incubated at 37°C for 2 hours and washed in PBST. After addition of goat antibody against human x chain labelled with alkaline phosphatase (Tago Company) (100 μl/well), the incubation was continued at 37°C for 2 hours, and the plate was washed in PBST. To the plate was added p-nitrophenyl phosphate (1 mg/ml) as a substrate for the colour reaction and allowed to react for 10 minutes at room temperature, and the $OD_{405}$ of the reaction mixture was measured.

Positive cells that expressed x chain are listed in the following table.

## Table

| | $OD_{405}$ | |
| Clones | supernatant | 1/4 diluted supernatant |
|---|---|---|
| 1A3 | 1.46 | 0.98 |
| 3D2 | 0.89 | 0.43 |
| 4B5 | 0.23 | 0.22 |
| 1D6 | 0.48 | 0.30 |

## Example 4

### Establishment of animal cells that express cDNAs encoding H and L chain of human anti-Ex-A antibody

pSV2neogFK1 containing x chain encoding gene was introduced into a mouse myeloma cell Sp2/0 in accordance with the procedure of Example 3-(6). The cell which is capable of expressing x chain encoding gene to a great extent was obtained and designated 3D6. An expression plasmid pSV2gptgFM1 for μ chain gene was additionally introduced into the cell, and a clone which produces an antibody capable of binding to the Ex-A was obtained and designated 3A6 (Fig. 9).

The production of the antibody was evaluated by ELISA and determined to be 23.6 μg/ml, which was higher than that of EB virus-transformant, FK-001. The clone 3A6 was stable for at least 1 month and continuously produced anti-Ex-A antibody.

## Example 5

### Cultivation of antibody-producing cell and purification of antibody

The clone 3A6 prepared in Example 4 was cultivated in a T-flask (Corning). The culture (500 ml) was salted out with a 50% $(NH_4)_2 SO_4$ saturated solution, and dialyzed against PBS(-). The dialyzed solution was chromatographed on Sephalose CL-4B for the purpose of partial purification, and fractions (protein = 5 mg) having the Ex-A-binding activity was collected. The IgM produced by 3A6 was proved to be a protein having a molecular weight of about 105,000, and to consist of H chain (Mw = about 80,000) and L chain (Mw = about 25,000). The affinity property of the antibody produced by said cell was identical with that of the antibody produced by the human anti-Ex-A antibody-producing cell, FK-001.

## Claims

1. A gene encoding an antibody to an exotoxin of Pseudomonas aeruginosa.

2. A gene of Claim 1 where V region of the H chain of the antibody has an amino acid sequence (vii) or (viii) described below:

(vii) MetSerValSerPheLeuIlePheLeuProValLeuGlyLeuProTrpGlyValLeuSer GlnValGlnLeuGlnGlnSer-GlyProGlyLeuValLysProSerGlnThrLeuSerLeu ThrCysAlaIleSerGlyAspSerValSerSerAsnSerValSerTrpAsnTrpIleArg GlnSerProSerArgGlyLeuGluTrpLeuGlyArgThrTyrTyrArgSerLysTrpTyr AsnAspSerAlaValSerValLysSerArgIleThrIleAsnProAspThrSerLysAsn GlnPheSerLeuGlnLeuAsnSerValThrProGluAspThrAlaValTyrTyrCysVal ArgAlaArgProGlyThrThr-GlyGlyGlyMetAspValTrpGlyGlnGlyThrThrVal ThrValSerSer,or

(viii) GlnValGlnLeuGlnGlnSerGlyProGlyLeuValLysProSerGlnThrLeuSerLeu ThrCysAlaIleSer-GlyAspSerValSerSerAsnSerValSerTrpAsnTrpIleArg GlnSerProSerArgGlyLeuGluTrpLeuGlyArgThrTyrTyrArgSerLysTrpTyr AsnAspSerAlaValSerValLysSerAr-gIleThrIleAsnProAspThrSerLysAsn GlnPheSerLeuGlnLeuAsnSerValThrProGluAspThrAlaValTyrTyrCysVal Ar-gAlaArgProGlyThrThrGlyGlyGlyMetAspValTrpGlyGlnGlyThrThrVal ThrValSerSer

3. A gene of Claim 1 wherein a gene encoding V region of the H chain of the antibody has a base sequence (i), (ii), or (iii) described below:

(i)

```
        10         -20           30          40          50          60
ATGTCTGTCT CCTTCCTCAT CTTCCTGCCC GTGCTGGGCC TCCCATGGGG TCAGTGTCAG
TACAGACAGA GGAAGGAGTA GAAGGACGGG CACGACCCGG AGGGTACCCC AGTCACAGTC

        70          80           90         100         110         120
GGAGATGCCG TATTCACAGC AGCATTCACA GACTGAGGGG TGTTTCACTT TGCTGTTTCC
CCTCTACGGC ATAAGTGTCG TCGTAAGTGT CTGACTCCCC ACAAAGTGAA ACGACAAAGG

       130         140          150         160          170        180
TTTTGTCTCC AGGTGTCCTG TCACAGGTAC AGCTGCAGCA GTCAGGTCCA GGACTGGTGA
AAAACAGAGG TCCACAGGAC AGTGTCCATG TCGACGTCGT CAGTCCAGGT CCTGACCACT

       190         200          210         220         230         240
AGCCCTCGCA GACCCTCTCA CTCACCTGTG CCATCTCCGG GGACAGTGTC TCTAGCAACA
TCGGGAGCGT CTGGGAGAGT GAGTGGACAC GGTAGAGGCC CCTGTCACAG AGATCGTTGT

       250          260         270         280         290         300
GTGTTTCTTG GAACTGGATC AGGCAGTCCC CATCGAGAGG CCTTGAGTGG CTGGGAAGGA
CACAAAGAAC CTTGACCTAG TCCGTCAGGG GTAGCTCTCC GGAACTCACC GACCCTTCCT

       310         320          330         340         350          360
CATACTACAG GTCCAAGTGG TATAATGATT CTGCAGTATC TGTGAAAAGT CGAATAACCA
GTATGATGTC CAGGTTCACC ATATTACTAA GACGTCATAG ACACTTTTCA GCTTATTGGT

       370         380          390         400         410         420
TCAACCCAGA CACATCCAAG AACCAGTTCT CCCTGCAGCT GAACTCTGTG ACTCCCGAGG
AGTTGGGTCT GTGTAGGTTC TTGGTCAAGA GGGACGTCGA CTTGAGACAC TGAGGGCTCC

       430         440          450         460         470         480
ACACGGCTGT GTATTACTGT GTAAGAGCCC GCCCCGGTAC GACCGGGGGT GGTATGGACG
TGTGCCGACA CATAATGACA CATTCTCGGG CGGGGCCATG CTGGCCCCCA CCATACCTGC

       490         500         510
TCTGGGGCCA AGGGACCACG GTCACCGTCT CCTCAG    , or
AGACCCCGGT TCCCTGGTGC CAGTGGCAGA GGAGTC
```

(ii)

```
        10          20           30          40          50          60
ATGTCTGTCT CCTTCCTCAT CTTCCTGCCC GTGCTGGGCC TCCCATGGGG TGTCCTGTCA
TACAGACAGA GGAAGGAGTA GAAGGACGGG CACGACCCGG AGGGTACCCC ACAGGACAGT

        70          80           90         100         110         120
CAGGTACAGC TGCAGCAGTC AGGTCCAGGA CTGGTGAAGC CCTCGCAGAC CCTCTCACTC
GTCCATGTCG ACGTCGTCAG TCCAGGTCCT GACCACTTCG GGAGCGTCTG GGAGAGTGAG

       130         140          150         160         170         180
ACCTGTGCCA TCTCCGGGGA CAGTGTCTCT AGCAACAGTG TTTCTTGGAA CTGGATCAGG
TGGACACGGT AGAGGCCCCT GTCACAGAGA TCGTTGTCAC AAAGAACCTT GACCTAGTCC

       190         200          210         220         230         240
CAGTCCCCAT CGAGAGGCCT TGAGTGGCTG GGAAGGACAT ACTACAGGTC CAAGTGGTAT
GTCAGGGGTA GCTCTCCGGA ACTCACCGAC CCTTCCTGTA TGATGTCCAG GTTCACCATA
```

```
           250       260       270       280       290       300
AATGATTCTG CAGTATCTGT GAAAAGTCGA ATAACCATCA ACCCAGACAC ATCCAAGAAC
TTACTAAGAC GTCATAGACA CTTTTCAGCT TATTGGTAGT TGGGTCTGTG TAGGTTCTTG

           310       320       330       340       350       360
CAGTTCTCCC TGCAGCTGAA CTCTGTGACT CCCGAGGACA CGGCTGTGTA TTACTGTGTA
GTCAAGAGGG ACGTCGACTT GAGACACTGA GGGCTCCTGT GCCGACACAT AATGACACAT

           370       380       390       400       410       420
AGAGCCCGCC CCGGTACGAC CGGGGGTGGT ATGGACGTCT GGGGCCAAGG GACCACGGTC
TCTCGGGCGG GGCCATGCTG GCCCCCACCA TACCTGCAGA CCCCGGTTCC CTGGTGCCAG

           430
ACCGTCTCCT CAG
TGGCAGAGGA GTC  , or
```

(iii)

```
            10        20        30        40        50        60
CAGGTACAGC TGCAGCAGTC AGGTCCAGGA CTGGTGAAGC CCTCGCAGAC CCTCTCACTC
GTCCATGTCG ACGTCGTCAG TCCAGGTCCT GACCACTTCG GGAGCGTCTG GGAGAGTGAG

            70        80        90       100       110       120
ACCTGTGCCA TCTCCGGGGA CAGTGTCTCT AGCAACAGTG TTTCTTGGAA CTGGATCAGG
TGGACACGGT AGAGGCCCCT GTCACAGAGA TCGTTGTCAC AAAGAACCTT GACCTAGTCC

           130       140       150       160       170       180
CAGTCCCCAT CGAGAGGCCT TGAGTGGCTG GGAAGGACAT ACTACAGGTC CAAGTGGTAT
GTCAGGGGTA GCTCTCCGGA ACTCACCGAC CCTTCCTGTA TGATGTCCAG GTTCACCATA

           190       200       210       220       230       240
AATGATTCTG CAGTATCTGT GAAAAGTCGA ATAACCATCA ACCCAGACAC ATCCAAGAAC
TTACTAAGAC GTCATAGACA CTTTTCAGCT TATTGGTAGT TGGGTCTGTG TAGGTTCTTG

           250       260       270       280       290       300
CAGTTCTCCC TGCAGCTGAA CTCTGTGACT CCCGAGGACA CGGCTGTGTA TTACTGTGTA
GTCAAGAGGG ACGTCGACTT GAGACACTGA GGGCTCCTGT GCCGACACAT AATGACACAT

           310       320       330       340       350       360
AGAGCCCGCC CCGGTACGAC CGGGGGTGGT ATGGACGTCT GGGGCCAAGG GACCACGGTC
TCTCGGGCGG GGCCATGCTG GCCCCCACCA TACCTGCAGA CCCCGGTTCC CTGGTGCCAG

           370
ACCGTCTCCT CAG
TGGCAGAGGA GTC
```

4. A gene of Claim 1 wherein V region of the L chain of the antibody has an amino acid sequence (ix) or (x) described below:

(ix)    MetValLeuGlnThrGlnValPheIleSerLeuLeuLeuTrpIleSerGlyAlaTyrGly    AspIleValMetThrGlnSer-ProAspSerLeuAlaValSerLeuGlyGluArgAlaThr

IleAsnCysLysSerSerGlnSerValLeuTyrSerSerAsnAsnLysAsnTyrLeuAla    TrpTyrGlnGlnLysProGlyGln-ProProLysLeuLeuIleTyrTrpAlaSerThrArg

GluSerGlyValProAspArgPheSerGlySerGlySerGlyThrAspPheThrLeuThr    IleSerSerLeuGlnAlaGluAspValAlaVal-TyrTyrCysGlnGlnTyrTyrSerThr ProArgThrPheGlyGlnGlyThrLysValGluIleLys ,or

(x) AspIleValMetThrGlnSerProAspSerLeuAlaValSerLeuGlyGluArgAlaThr  IleAsnCysLysSerSerGlnSer-ValLeuTyrSerSerAsnAsnLysAsnTyrLeuAla
TrpTyrGlnGlnLysProGlyGlnProProLysLeuLeuIleTyrTrpAlaSerThrArg  GluSerGlyValProAspArgPheSerGlySer-GlySerGlyThrAspPheThrLeuThr  IleSerSerLeuGlnAlaGluAspValAlaValTyrTyrCysGlnGlnTyrTyrSerThr  ProArg-ThrPheGlyGlnGlyThrLysValGluIleLys

5. A gene of Claim 1 wherein a gene encoding V region of the L chain of the antibody has a base sequence (iv), (v), or (vi) described below:

(iv)

```
          10         20         30         40         50         60
ATGGTGTTGC AGACCCAGGT CTTCATTTCT CTGTTGCTCT GGATCTCTGG TGAGGAATTA
TACCACAACG TCTGGGTCCA GAAGTAAAGA GACAACGAGA CCTAGAGACC ACTCCTTAAT

          70         80         90        100        110        120
AAAAGTGCCA CAGTCTTTTC AGAGTAATAT CTGTGTAGAA ATAAAAAAAA TTAAGATATA
TTTTCACGGT GTCAGAAAAG TCTCATTATA GACACATCTT TATTTTTTT AATTCTATAT

         130        140        150        160        170        180
GTTGGAAATA ATGACTATTT CCAATATGGA TCCAATTATC TGCTGACTTA TAATACTACT
CAACCTTTAT TACTGATAAA GGTTATACCT AGGTTAATAG ACGACTGAAT ATTATGATGA

         190        200        210        220        230        240
AGAAAGCAAA TTTAAATGAC ATATTTCAAT TATATCTGAG ACAGCGTGTA TAAGTTTATG
TCTTTCGTTT AAATTTACTG TATAAAGTTA ATATAGACTC TGTCGCACAT ATTCAAATAC

         250        260        270        280        290        300
TATAATCATT GTCCATTCCT GACTACAGGT GCCTACGGGG ACATCGTGAT GACCCAGTCT
ATATTAGTAA CAGGTAAGGA CTGATGTCCA CGGATGCCCC TGTAGCACTA CTGGGTCAGA

         310        320        330        340        350        360
CCAGACTCCC TGGCTGTGTC TCTGGGCGAG AGGGCCACCA TCAACTGCAA GTCCAGCCAG
GGTCTGAGGG ACCGACACAG AGACCCGCTC TCCCGGTGGT AGTTGACGTT CAGGTCGGTC
```

26

```
            370        380         390          400          410        420
     AGTGTTTTAT ACAGCTCCAA CAATAAGAAC TACTTAGCTT GGTACCAGCA GAAACCAGGA
     TCACAAAATA TGTCGAGGTT GTTATTCTTG ATGAATCGAA CCATGGTCGT CTTTGGTCCT

            430        440         450          460          470        480
     CAGCCTCCTA AGCTGCTCAT TTACTGGGCA TCTACCCGGG AATCCGGGGT CCCTGACCGA
     GTCGGAGGAT TCGACGAGTA AATGACCCGT AGATGGGCCC TTAGGCCCCA GGGACTGGCT

            490        500         510          520          530        540
     TTCAGTGGCA GCGGGTCTGG GACAGATTTC ACTCTCACCA TCAGCAGCCT GCAGGCTGAA
     AAGTCACCGT CGCCCAGACC CTGTCTAAAG TGAGAGTGGT AGTCGTCGGA CGTCCGACTT

            550        560         570          580          590        600
     GATGTGGCAG TTTATTACTG TCAGCAATAT TATAGTACTC CTCGTACGTT CGGCCAAGGG
     CTACACCGTC AAATAATGAC AGTCGTTATA ATATCATGAG GAGCATGCAA GCCGGTTCCC

            610
     ACCAAGGTGG AAATCAAAC
     TGGTTCCACC TTTAGTTTG  ,or
```

(v)

```
            10         20          30          40           50         60
     ATGGTGTTGC AGACCCAGGT CTTCATTTCT CTGTTGCTCT GGATCTCTGG TGCCTACGGG
     TACCACAACG TCTGGGTCCA GAAGTAAAGA GACAACGAGA CCTAGAGACC ACGGATGCCC

            70         80          90          100          110        120
     GACATCGTGA TGACCCAGTC TCCAGACTCC CTGGCTGTGT CTCTGGGCGA GAGGGCCACC
     CTGTAGCACT ACTGGGTCAG AGGTCTGAGG GACCGACACA GAGACCCGCT CTCCCGGTGG

            130        140         150          160          170        180
     ATCAACTGCA AGTCCAGCCA GAGTGTTTTA TACAGCTCCA ACAATAAGAA CTACTTAGCT
     TAGTTGACGT TCAGGTCGGT CTCACAAAAT ATGTCGAGGT TGTTATTCTT GATGAATCGA

            190        200         210          220          230        240
     TGGTACCAGC AGAAACCAGG ACAGCCTCCT AAGCTGCTCA TTTACTGGGC ATCTACCCGG
     ACCATGGTCG TCTTTGGTCC TGTCGGAGGA TTCGACGAGT AAATGACCCG TAGATGGGCC

            250        260         270          280          290        300
     GAATCCGGGG TCCCTGACCG ATTCAGTGGC AGCGGGTCTG GACAGATTT CACTCTCACC
     CTTAGGCCCC AGGGACTGGC TAAGTCACCG TCGCCCAGAC CCTGTCTAAA GTGAGAGTGG

            310        320         330          340          350        360
     ATCAGCAGCC TGCAGGCTGA AGATGTGGCA GTTTATTACT GTCAGCAATA TTATAGTACT
     TAGTCGTCGG ACGTCCGACT TCTACACCGT CAAATAATGA CAGTCGTTAT AATATCATGA

            370        380         390          400
     CCTCGTACGT TCGGCCAAGG GACCAAGGTG GAAATCAAAC
     GGAGCATGCA AGCCGGTTCC CTGGTTCCAC CTTTAGTTTG  ,or
```

(vi)

```
        10         20         30         40         50         60
GACATCGTGA TGACCCAGTC TCCAGACTCC CTGGCTGTGT CTCTGGGCGA GAGGGCCACC
CTGTAGCACT ACTGGGTCAG AGGTCTGAGG GACCGACACA GAGACCCGCT CTCCCGGTGG

        70         80         90        100        110        120
ATCAACTGCA AGTCCAGCCA GAGTGTTTTA TACAGCTCCA ACAATAAGAA CTACTTAGCT
TAGTTGACGT TCAGGTCGGT CTCACAAAAT ATGTCGAGGT TGTTATTCTT GATGAATCGA

       130        140        150        160        170        180
TGGTACCAGC AGAAACCAGG ACAGCCTCCT AAGCTGCTCA TTTACTGGGC ATCTACCCGG
ACCATGGTCG TCTTTGGTCC TGTCGGAGGA TTCGACGAGT AAATGACCCG TAGATGGGCC

       190        200        210        220        230        240
GAATCCGGGG TCCCTGACCG ATTCAGTGGC AGCGGGTCTG GGACAGATTT CACTCTCACC
CTTAGGCCCC AGGGACTGGC TAAGTCACCG TCGCCCAGAC CCTGTCTAAA GTGAGAGTGG

       250        260        270        280        290        300
ATCAGCAGCC TGCAGGCTGA AGATGTGGCA GTTTATTACT GTCAGCAATA TTATAGTACT
TAGTCGTCGG ACGTCCGACT TCTACACCGT CAAATAATGA CAGTCGTTAT AATATCATGA

       310        320        330        340
CCTCGTACGT TCGGCCAAGG GACCAAGGTG GAAATCAAAC C
GGAGCATGCA AGCCGGTTCC CTGGTTCCAC CTTTAGTTTG G
```

6. A gene of Claim 3, wherein the H chain is μ chain.

7. A gene of Claim 5, wherein the L chain is x chain.

8. A recombinant plasmid or virus which comprises integrated therein a gene encoding an antibody to an exotoxin of Pseudomonas aeruginosa.

9. A recombinant plasmid or virus of Claim 8 wherein a gene encoding V region of the H chain of the antibody has the base sequence (i), (ii), or (iii).

10. A recombinant plasmid or virus of Claim 8 wherein a gene encoding V region of the L chain of the antibody has the base sequence (iv), (v), or (vi).

11. A recombinant plasmid of Claim 8, 9 or 10 wherein a vector employed is pSV2gpt or pSV2neo.

12. A transformed host cell which contains a recombinant plasmid or virus which comprises integrated therein a gene encoding an antibody to an exotoxin of Pseudomonasaeruginosa.

13. A transformed host cell of Claim 12, which contains a recombinant plasmid or virus which comprises integrated therein a gene encoding an antibody to an exotoxin of Pseudomonasaeruginosa. wherein a gene encoding V region of the H chain of the antibody has a base sequence (i), (ii), or (iii) described in Claim 4.

14. A transformed host cell which contains a recombinant plasmid or virus which comprises integrated therein a gene encoding an antibody to an exotoxin of Pseudomonas aeruginosa, wherein a gene encoding V region of the L chain of the antibody has the nucleotide sequence (iv), (v), or (vi).

15. A transformed host cell of any one of Claims 12 to 14 wherein the host cell is Escherichia coli.

16. A transformed host cell of any one of Claims 12 to 14 wherein the host cell is a mammalian cell.

17. A transformed host cell of any one of Claims 12 to 14 wherein the host cell is Namalwa cell or subspecies thereof.

18. A recombinant antibody wherein V region of the H chain of the antibody has the amino acid sequence (vii) or (viii) and V region of the L chain of the antibody has the amino acid sequence (ix) or (x).

19. A transformed cell DHI (pSV2neogFK1) FERM BP-1548).

20. A transformed cell DHI (pSV2gptgFM1) (FERM BP-1550).

21. A transformed cell DHI (pSV2neoSV1FK1-1) (FERM BP-1549).

22. A process for preparing human monoclonal antibody to an exotoxin of Pseudomonas aeruginosa which comprises culturing recombinant transformants capable of expressing a gene encoding human anti-exotoxin-antibody in an appropriate culture medium and recovering produced antibody from the culture medium.

23. A process of Claim 22 wherein the host cell is a strain of genus Escherichia.

24. A process of Claim 22 wherein the host cell is B lymphocyte derived from mammalian.

25. A process of Claim 22 wherein the host cell is Namalwa cell or subspecies thereof.

26. A process of Claim 22 wherein the host cell is mouse myeloma cell.

27. A process of Claim 22 wherein the recombinant plasmid is pSV2gptgFM1.

28. A process of Claim 22 wherein the recombinant plasmid is pSV2neogFK1.

29. A process of Claim 22 wherein the recombinant plasmid is a combination of pSV2gptgFM1 and pSV2neogFK1.

30. A process of Claim 22 wherein the gene encoding the H chain of the monoclonal antibody contains in its V region a gene having the nucleotide sequence (i), (ii), or (iii) and the gene encoding the L chain of the monoclonal antibody contains in its V region a gene having the base sequence (iv), (v), or (vi).

Fig. 1

A)

L VDJ

Cμ

B)

L VJ

Ck

B:BamHI    H:HindⅢ    S: SacI

0 270 077

Fig.2 - 1

```
          10        20        30        40        50        60
GGATCTTTCA GCAGTTTGTA GTTTTAGAGC TTCTAAGTTG ACTTCTGTCT TTTCTAATTC

          70        80        90       100       110       120
ATACAATTAC ACATTCTGTG ATGATATTTT TGGCTCTTGA TTTACATTGG GTACTTTCAC

         130       140       150       160       170       180
AACCCACTGC TCATGAAATT TGCTTTTGTA CTCACTGGTT GTTTTTGCAT AGGCCCCTCC

         190       200       210       220       230       240
AGGCCACGAC CAGCTGTTTG GATTTTATAA ACGGGCCGTT TGCATTGTGA ACTGAGCTAC

         250       260       270       280       290       300
AACAGGCAGG CAGGGGCAGC AAGATGGTGT TGCAGACCCA GGTCTTCATT TCTCTGTTGC

         310       320       330       340       350       360
TCTGGATCTC TGGTGAGGAA TTAAAAAGTG CCACAGTCTT TTCAGAGTAA TATCTGTGTA

         370       380       390       400       410       420
GAAATAAAAA AAATTAAGAT ATAGTTGGAA ATAATGACTA TTTCCAATAT GGATCCAATT

         430       440       450       460       470       480
ATCTGCTGAC TTATAATACT ACTAGAAAGC AAATTTAAAT GACATATTTC AATTATATCT

         490       500       510       520       530       540
GAGACAGCGT GTATAAGTTT ATGTATAATC ATTGTCCATT CCTGACTACA GGTGCCTACG
```

0 270 077

Fig. 2-2

```
        550        560        570        580        590        600
GGGACATCGT GATGACCCAG TCTCCAGACT CCCTGGCTGT GTCTCTGGGC GAGAGGGCCA

        610        620        630        640        650        660
CCATCAACTG CAAGTCCAGC CAGAGTGTTT TATACAGCTC CAACAATAAG AACTACTTAG

        670        680        690        700        710        720
CTTGGTACCA GCAGAAACCA GGACAGCCTC CTAAGCTGCT CATTTACTGG GCATCTACCC

        730        740        750        760        770        780
GGGAATCCGG GGTCCCTGAC CGATTCAGTG GCAGCGGGTC TGGGACAGAT TTCACTCTCA

        790        800        810        820        830        840
CCATCAGCAG CCTGCAGGCT GAAGATGTGG CAGTTTATTA CTGTCAGCAA TATTATAGTA

        850        860        870        880        890        900
CTCCTCGTAC GTTCGGCCAA GGGACCAAGG TGGAAATCAA ACGTGAGTAG AATTTAAATT

        910        920        930        940        950        960
TTGCTTCCTC AGTTGTCTGT GTCTTCTGCT CCCTGTGTCT ATGAAGTGAT CTATAAGCTG

        970        980        990       1000       1010       1020
ACTCTGCAAT CAGCCTCTGA TTTCCTTCAG GGAAAAGATA AAGATAAGTC TGTAGTCAAG

       1030       1040       1050       1060       1070       1080
CTCGAGAATT GATTGCACAT TTTCTTTGAA GAGCAAGCAA GATTCAGTCA TTGGGTGAGA
```

## Fig.2 -3

```
  1090        1100        1110        1120        1130        1140
ATAACTTGTC  TAAGTAATAG  CTTCAGAAAT  GTCCTGGGGA  ACATAACATG  TTCTGGACAG

  1150        1160        1170        1180        1190        1200
AGCCTTGGTC  AATTGTCAGA  AAGGGAGTTT  TTGTATAGGA  GGGAAGTTAA  GAGGAAAAAT

  1210        1220        1230        1240        1250        1260
TGTGCACAGT  GATACAAATA  ATGCCACTAA  GGGGAAGAGA  ACAGAAACGT  AATGGGCGCT

  1270        1280        1290        1300        1310        1320
GAGCTGGGAA  AACCAGGGAG  AAGACTGATT  TATTAGAGAT  TTCAGAAATA  AAATTCACAT

  1330        1340        1350        1360        1370
TCATTATGAT  ATCTCATTAG  TGAAAATTTC  CATTAGGGGA  TTGTAAATAA  TTT
```

0 270 077

## Fig.3 -1

```
        10          20          30          40          50          60
AGGGCCCCGG CTCTTCAATG AGCCATCTCC GTCCCGGGGC CTTATATCAG CAAGTGACGC

        70          80          90         100         110         120
ACACAGGCAA ATGCCAGGGT GTGGTTTCCT GTTTAAATGT AGCCTCCCCC GCTGCAGAAC

       130         140         150         160         170         180
TGCAGAGCCT GCTGAATTCT GGCTGACCAG GGCAGTCACC AGAGCTCCAG ACAATGTCTG

       190         200         210         220         230         240
TCTCCTTCCT CATCTTCCTG CCCGTGCTGG GCCTCCCATG GGGTCAGTGT CAGGGAGATG

       250         260         270         280         290         300
CCGTATTCAC AGCAGGATTC ACAGACTGAG GGGTGTTTCA CTTTGCTGTT TCCTTTTGTC

       310         320         330         340         350         360
TCCAGGTGTC CTGTCACAGG TACAGCTGCA GCAGTCAGGT CCAGGACTGG TGAAGCCCTC

       370         380         390         400         410         420
GCAGACCCTC TCACTCACCT GTGCCATCTC CGGGGACAGT GTCTCTAGCA ACAGTGTTTC

       430         440         450         460         470         480
TTGGAACTGG ATCAGGCAGT CCCCATCGAG AGGCCTTGAG TGGCTGGGAA GGACATACTA

       490         500         510         520         530         540
CAGGTCCAAG TGGTATAATG ATTCTGCAGT ATCTGTGAAA AGTCGAATAA CCATCAACCC
```

0 270 077

## Fig.3 -2

```
        550        560        570        580        590        600
AGACACATCC AAGAACCAGT TCTCCCTGCA GCTGAACTCT GTGACTCCCG AGGACACGGC

        610        620        630        640        650        660
TGTGTATTAC TGTGTAAGAG CCCGCCCCGG TACGACCGGG GGTGGTATGG ACGTCTGGGG

        670        680        690        700
CCAAGGGACC ACGGTCACCG TCTCCTCAGG TAAGAATGGC CA
```

0 270 077

Fig. 4

# Fig.5

## Fig.6

### K chain cDNA

## Fig.8

### K chain cDNA

## Fig. 9

Dilution of Culture Supernatant

## Fig.7

① DNA

Genomic DNA    Subcloning into pUC 19
Recovering EcoR I-
Bam HI fragment

c DNA    Subcloning into pUC 19
EcoRI digestion

② Ligation of EcoRI sites by T4 ligase

Homologous recombi-
nation at L region

③ Plasmid obtained after E.coli JM 83 transformation
with above DNA (②)

□ : Exon (L or V region)    E : EcoRI    H : Hind Ⅲ

— : Intron    B : Bam HI    Bg : Bgl Ⅱ

☐ : pUC19 multi-cloning site    P : Pst I

● : Immunoglobulin promoter